# EUROPEAN PATENT SPECIFICATION

(11) **EP 0 626 099 B1**
(45) Date of publication and mention of the grant of the patent: **13.11.2002**
(21) Application number: 93905940.8
(22) Date of filing: 12.02.1993
(51) Int. Cl.: H01L 21/768

(54) **METHOD OF MANUFACTURING A HIGH DENSITY ELECTRONIC CIRCUIT MODULE**
VERFAHREN ZUR HERSTELLUNG EINES ELEKTRONISCHEN SCHALTUNGSMODULS MIT HOHER INTEGRATIONSDICHTE
PROCEDE DE FABRICATION D'UN MODUL DE CIRCUITS ELECTRONIQUES A HAUTE DENSITE

(30) Priority: 13.02.1992 US 834849; 24.04.1992 US 874588
(43) Date of publication of application: 30.11.1994
(60) Divisional application: 02076327.2
(73) Proprietor: KOPIN CORPORATION, Taunton, MA 02780 (US)
(72) Inventor: SPITZER, Mark, B., Sharon, MA 02067 (US); JACOBSEN, Jeffrey, Hollister, CA 95023 (US); VU, Duy-Phach, Taunton, MA 02780 (US); DINGLE, Brenda, Mansfield, MA 02048 (US); CHEONG, Ngwe, Boston, MA 02116 (US)
(74) Representative: Greenwood, John David
(86) International application number: PCT/US93/01322
(87) International publication number: WO 93/016491

(56) References cited:
- ELEKTRONIKER, no. 10, October 1989, Aaran U. K|NIG: "Dreidimensionale Integration" pages 79-82,
- SIEMENS COMPONENTS, vol. 27, no. 2, March/April 1989, Berlin u. M nchen E. HOFMEISTER. "Mikroelektro- nik 2000" pages 54-58,
- US-A-4816420

## Description

### Background of the Invention

The development of new portable electronic products, such as the laptop computer, is currently of great worldwide interest. Miniaturization of the various component systems (memories, displays, and so forth) for such products requires that the necessary circuits be packed in as small a volume as possible. Packing circuits into a small volume also reduces parasitic capacitance and improves signal propagation time between circuits. One approach to this requirement is to increase the scale of integration in order to obtain all of the required functions from a circuit made from a single wafer. Unfortunately, efforts to create full-wafer circuitry have encountered unacceptable yield losses owing to the large circuit size. In the specific area of active matrix displays, a similar problem results in attempting the scale-up of the display size to and beyond the 256K pixel level.

Active matrix (AM) displays generally consist of flat-panels consisting of liquid crystals or electroluminescent materials which are switched "on" and "off" by electric fields emanating from pixel electrodes charged by thin-film transistors (TFT's) co-located with each liquid crystal or electroluminescent pixel area. These AM displays are expected to supplant cathode ray tube (CRT) technology and provide a more highly defined television picture or data display. The primary advantage of the active matrix approach, using TFT's, is the elimination of cross-talk between pixels, and the excellent grey scale that can be attained with TFT-compatible liquid crystal displays (LCD's).

Flat panel displays employing LCD's generally include five different layers: a white light source layer, a first polarizing filter layer that is mounted on one side of a circuit panel on which the TFT's are arrayed to form pixels, a filter plate layer containing at least three primary colors arranged into pixels, and finally a second polarizing filter layer. A volume between the circuit panel and the filter plate is filled with a liquid crystal material. This material rotates the polarization of light passing through it when an appropriate electric field is applied across it. Thus, when a particular pixel electrode of the display is charged up by an associated TFT, the liquid crystal material rotates polarized light being transmitted through the material so that it will pass through the second polarizing filter and be seen by the viewer.

The primary approach to TFT formation over the large areas required for flat panel displays has involved the use of films of amorphous silicon which has previously been developed for large-area photovoltaic devices. Although the TFT approach has proven to be feasible, the use of amorphous silicon compromises certain aspects of the panel performance. For example, amorphous silicon TFT's lack the frequency response needed for large area displays due to the low electron mobility inherent in amorphous material. Thus, the use of amorphous silicon limits display speed, and is also unsuitable for the fast logic needed to drive the display.

Owing to the limitations of amorphous silicon, other alternative materials are being considered, such as, polycrystalline silicon, or laser recrystallized silicon. Thin films, less than about 0.4 microns, of these materials are usually formed on glass which generally restricts further circuit processing to low temperatures.

The formation of large active-matrix displays is hampered by the unavailability of large-area single crystal Si material. Thus the conventional approach is to use thin-film amorphous (Á-Si) or polycrystalline Si (poly-Si) wafers. The required number of thin-film transistors (TFT's), combined with the large number of driver circuits and the thin-film material defects inherent in Á-Si or poly-Si, leads to unacceptable yield and quality problems when the entire display is to be fabricated as a unit.

A need exists, therefore, for a relatively inexpensive way to reliably form hybrid high density electronic circuits, including active matrices, memories, and other devices, in a modular approach that permits small high-quality parts or circuits to be assembled into complete large-area high-quality complex devices.

EP-A1-0316 799 describes a semiconductor device having a semiconductor layer, in which an active device such as a MOS or bipolar transistor in the front surface area of the semiconductor layer, and a device for communicating the front and rear surfaces of the semiconductor layer is formed therein in connection with one of the electrodes of the active device and exposes to a groove formed in the rear surface of the semiconductor layer. A three-dimensional semiconductor device including at least two semiconductor devices (an upper and lower device) stacked one on another is also disclosed. This application states that the coupling of the upper and lower semiconductor devices is performed in almost the same way as that disclosed in the report "Promising New Fabrication Process Developed for Stacked LSI'S" of International Electron Devices Meeting Technical Digest, 1984, pp816, by M Yasumoto et al.

In this method, first, two two-layer gold-titanium (Au/Ti) alloy layers are formed on the two aluminum electrodes of the upper and lower devices, and then a polyimide layer is coated over the surface of the substrate up to the same height as that of the Au/Ti layer electrodes. Then, the coated surface of the substrate is treated by a plasma oxygen (O₂) etching to expose the Au/Ti electrodes and to make flat the surface of the polyimide layer and the Au/Ti electrodes in the same time.

On the other hand, at the rear surface of the first device an aluminum electrode integrally connected to a contact portion and another aluminum electrode are prepared, in advance, in the proper positions, and then two Au/Ti layer electrodes are formed on the two aluminum electrodes so as to coincide with the Au/Ti electrodes of the second device when the two devices are positioned to align to the desired position. Meanwhile an insulating film is applied on an insulating oxide film of the rear surface of the substrate. Then, a polyimide layer is coated over the rear surface of the substrate and the coated rear surface is also treated to a flat surface in the same manner as the flattened front surface of the second device as described above. The two flattened surfaces of the two devices are contacted with each other so as to contact the Au/Ti electrodes of the first device with the Au/Ti electrodes of the second device in the right position, and then the pairs of Au/Ti electrodes are securely connected to each other by the heat-pressure melting method, thereby coupling the first and second semiconductor devices.

When the two devices are connected with a sufficient strength by connecting only the electrode portions, dummy electrodes of the like may be further additionally formed on the contact surfaces of the two devices. This application goes on to state that this is one example for securely connecting the Au/Ti electrodes but that other proper methods may be used.

In the above described embodiment, the polyimide layers are said to effectively function for damping the stresses and giving the electric insulation. Further an etched groove in one of the substrates may be closed by filling with the polyimide material in a devised manufacturing process.
The method of the present invention is as claimed in claim 1.

### Brief Description of the Drawings

The following figures 1, 2A-B, 3, 4A-L, 5A-B, 6, 7A-D, 8A-B, 9A-B, 10A-B, 11A-D, 12A-B, 13A-B, 14, 15A-G, 16A-5, 18A-H, 19A-D, 20, 21, 22 and 23 depict examples not forming part of the invention

Fig. 1 is a perspective view of a high density circuit module in the form of an active matrix liquid crystal display (AMLCD).

Fig. 2A is a schematic illustrating how two six inch wafers can be used to form tiles for a 4 X 8 inch AMLCD.

Fig. 2B shows the tiles of Fig. 2A applied to a glass substrate for forming an AMLCD.

Fig. 3 is a circuit diagram illustrating the driver system for the AMLCD of Fig. 1.

Figs. 4A-4L is a preferred process flow sequence illustrating the fabrication of the a portion of the circuit panel for the AMLCD of Fig. 1.

Figs. 5A and 5B are cross-sectional schematic process views of a portion of the AMLCD.

Fig. 6 illustrates in a perspective view a preferred example of a system used for recrystallization.

Figs. 7A-7D is a process flow sequence illustrating transfer and bonding of a silicon an oxide (SOI) structure to a glass superstrate and removal of the substrate.

Figs. 8A and 8B is a process flow sequence illustrating an alternative transfer process in which a GeSi alloy is used as an intermediate etch step layer.

Figs. 9A and 9B is a process flow sequence illustrating another thin film tile isolate and transfer process used to form a pressure sensor or an array of such sensors.

Fig. 10A and 10B illustrate an alternate process to the process of Figs. 9A and 9B.

Figs. 11A-11D is a process flow sequence illustrating circuit transfer steps employed in the formation of a three-dimensional circuit.

Figs. 12A and 12B are graphs illustrating the drive current and transconductance of a MOSFET circuit surrounded by an adhesive and positioned on a glass substrate and a MOSFET circuit surrounded by air and positioned on a glass substrate respectively.

Figs. 13A and 13B is a process flow sequence illustrating the formation of electrical interconnections between layered devices.

Fig. 14 illustrates a shielding layer positioned in a layered structure for minimizing undesirable electrical interference between layered devices.

Figs. 15A-15G is a process flow sequence illustrating the fabrication of a 3-D double gate MOSFET device.

Figs. 16A-16J is a process flow sequence illustrating the fabrication of a 3-D double gate inverter.

Figs. 17A-17D is a process flow sequence illustrating the fabrication of a 3-D stacked inverter.

Figs. 18A-18H is a process flow sequence illustrating the fabrication of a vertical bipolar transistor.

Figs. 19A-19D illustrate the fabrication of a III-V circuit array.

Figs. 20 is a plan view of an XY addressable LED array mounted or stacked on a silicon substrate with an associated silicon electronic circuitry.

Fig. 21 is a schematic side view of an infrared to visible light converter example of the invention.

Fig. 22 is a side view of a pixel of a tri-color X-Y addressable LED array.

Fig. 23 is a plan view of the array of Fig. 22.

Figs. 24A-24C illustrate preferred examples of interconnecting 3D circuit stacks.

Figs. 25A-25C illustrate a preferred example of a thermally managed 3D stack.

### Details Description of the Invention

### I. Tiled Active Matrix Liquid Crystal Display

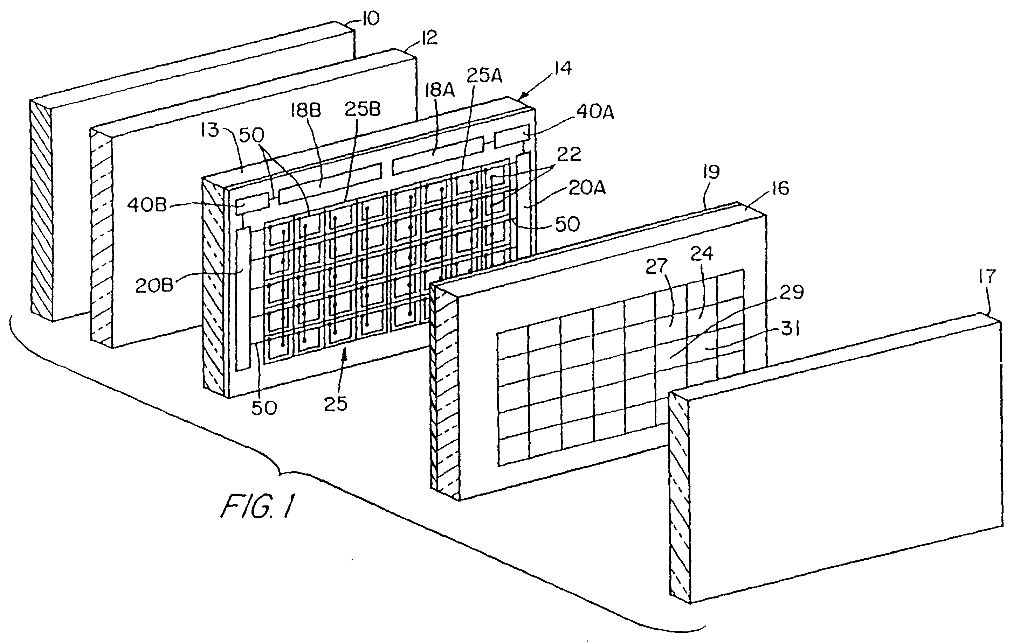

A preferred example not forming part of the invention for fabricating complex hybrid multi-function circuitry on common module substrates is illustrated in the context of an AMLCD, as shown in Fig. 1. The basic components of the AMLCD comprise a light source 10, such as a flat fluorescent or incandescent white lamp, or an electroluminescent lamp having white, or red, blue and green phosphors, a first polarizing filter 12, a circuit panel 14, an optional filter plate 16 and a second polarizing filter 17, which form a layered structure. Note: Filter plate 16 is not needed for a black and white display or where the red, green and blue colors are provided by the lamp at the appropriate pixel. A liquid crystal material 23, such as a twisted nematic is placed between the circuit panel 14 and the filter plate 16.

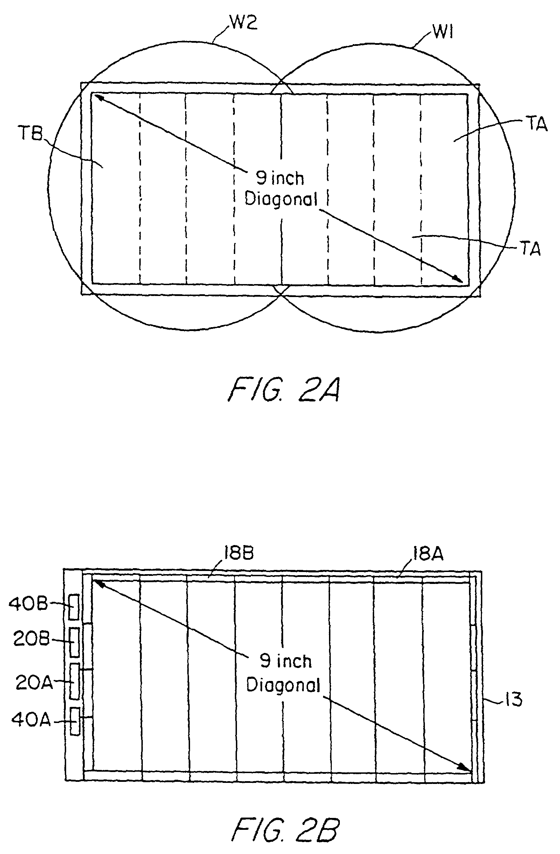

Circuit panel 14 consists of a transparent common module body 13 formed, for example, of glass upon which is transferred a plurality of common multifunction circuits comprising control logic circuits 40A and 40B and drive circuits 18A and 18B, 20A and 20B, and array circuit 25A and 25B. Preferably, the logic and drive circuits which require high speed operation are formed in tiles of x-Si. The array circuits may be formed in Á-Si material, or poly-Si, or preferably in x-Si, to achieve lower leakage in the resultant TFT's and, hence, better grey scale. Higher speed is also achieved in x-Si. A 4 x 8 inch (1 inch = 0.0254 m) active matrix LCD array can be formed from two standard 6-inch diameter Si wafers W1 and W2 as shown in Fig. 2A. Array circuit 25A is formed on wafer W1 and 1-inch by 4-inch tiles TA are transferred from the wafer W1 to the substrate 14. Note: The transfer may be accomplished using either a single or double transfer process, as will be described in detail below. Each tile is registered against another using micropositioning equipment and manipulators capable of micron scale accuracy. Similarly, tiles TB are transferred from wafer W2 to form array 25B on substrate or common module body 13 (See Fig. 2B).

Logic circuits 40A and 40B and drive circuits 18A, 18B, 20A, 20B are formed on other suitable substrates (not shown) and tiled and transferred in like manner to common substrate 13 and registered opposite the arrays 25A, 25B, as shown in Fig. 1. Conductive interconnections 50 are then made between the drive circuits and the individual pixels 22 and the logic control circuits 40A and 40B. In this manner, a 1280 by 1024 addressable array of pixels 22 are formed on the substrate 13 of circuit panel 14. Each pixel 22 is actuated by voltage from a respective drive circuit 18A or B on the X-axis and 20A or B on the Y-axis. The X and Y drive circuits are controlled by signals from control logic circuits 40A and B. Each pixel 19 produces an electric field in the liquid crystal material 23. disposed between the pixel and a counterelectrode (not shown) formed on the back side of the color filter plate 16.

The electric field formed by pixels 22 causes a rotation of the polarization of light being transmitted across the liquid crystal material that results in an adjacent color filter element being illuminated. The color filters of filter plate system 16 are arranged into groups of four filter elements, such as blue 24, green 31, red 27, and white 29. The pixels associated with filter elements can be selectively actuated to provide any desired color for that pixel group.

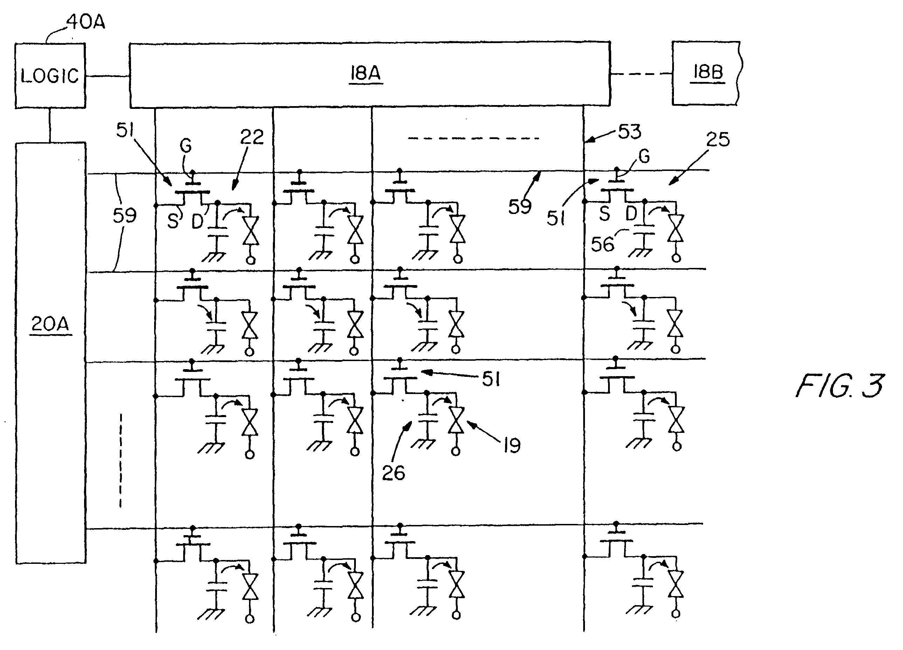

A typical drive and logic circuit that can be used to control the array pixels 22 is illustrated in Fig. 3. Drive circuit 18A receives an incoming signal from control logic 40A and sends a signal to each source electrode of a TFT 51 in one of the columns selected by logic circuit 40A through interconnect line 53. Y-drive circuit 20A controlled by logic circuit 40A energizes a row buss 59 extending perpendicular to column buss 53 and applies a voltage pulse to each gate G of TFT's 51 in a selected row. When a TFT has a voltage pulse on both its gate and source electrode current flows through an individual transistor 51, which charges capacitor 56 in a respective pixel 22. The capacitor 56 sustains a charge on the pixel electrode adjacent to the liquid crystal material (shown schematically at 19) until the next scan of the pixel array 25. Note: The various examples of the invention may, or may not, utilize capacitors 56 with each pixel depending upon the type of display desired.

### II. Transfer Processes

The array circuits 25A and 25B and logic 40A, 40B and drive circuits 18A, 18B may be formed and transferred by a number of processes. The basic steps in a single transfer process are: forming of a plurality of thin film Si circuits on Si substrates, dicing the thin film to form tiles, and transferring the tiles to a common module substrate by "tiling." Tiling involves the steps of transferring, registering the transferred tiles, and adhering the registered tiles. The Si substrates are then removed and the circuits on the tiles are interconnected.

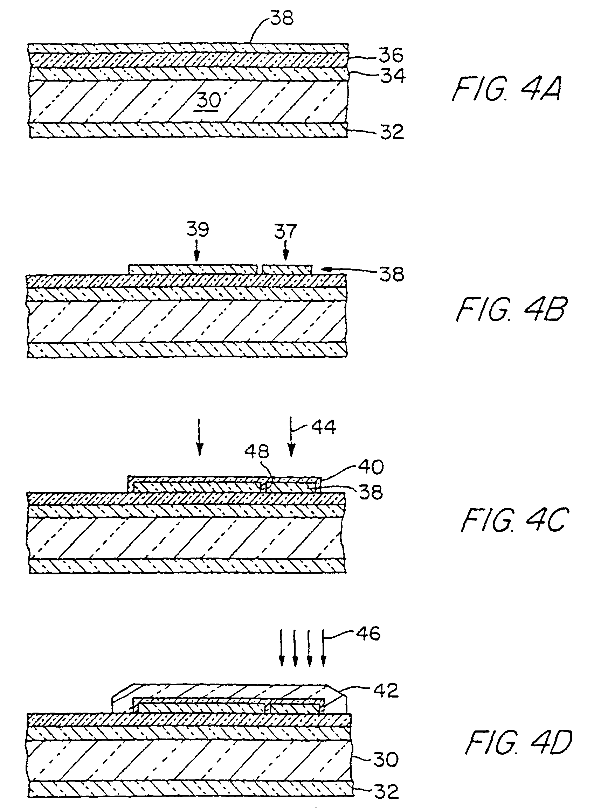

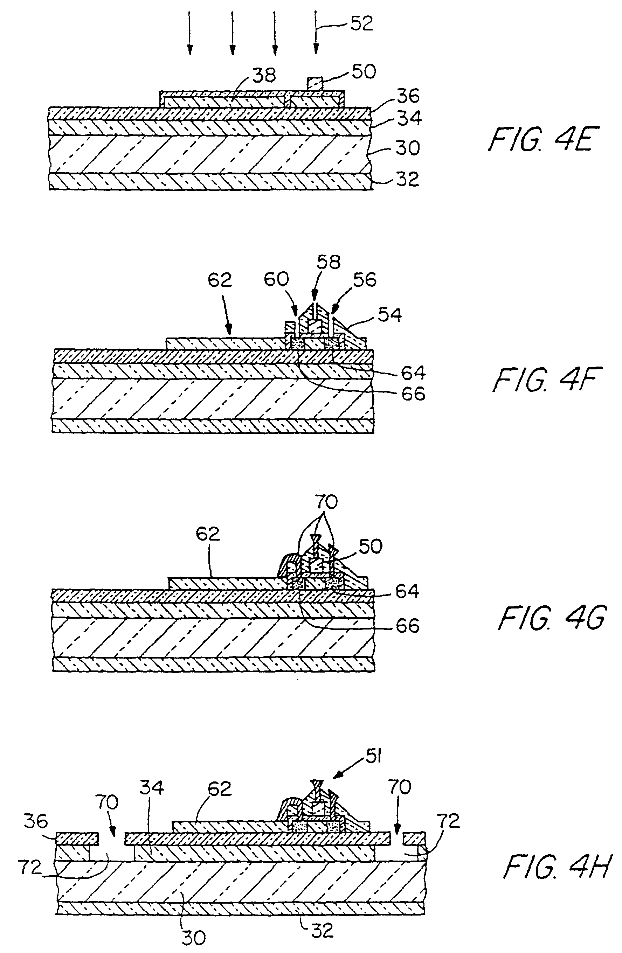

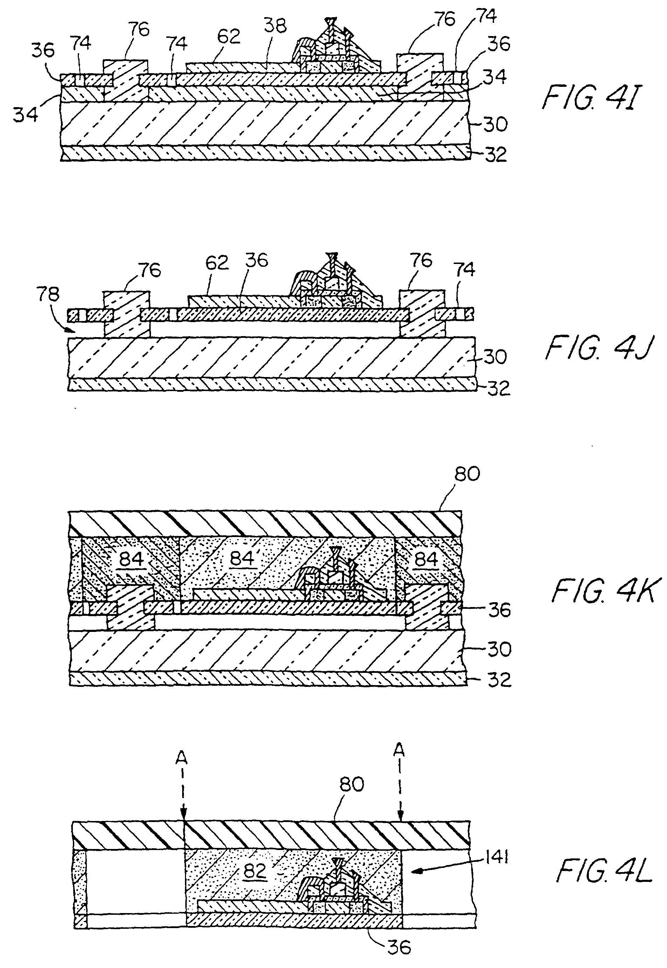

The double transfer approach, described in detail below in connection with Figs. 4A-4L is similar except that the Si-substrate is removed after dicing and the thin film is transferred to an intermediate transfer body or carrier before ultimate transfer to the common module body.

Assuming an Isolated Silicon Epitaxy (ISE) process is used, the first step is to form a thin-film precursor structure of silicon-on-insulator (SOI) film. An SOI structure, such as that shown in Fig. 4A, includes a substrate 32 of Si, a buffer layer 30, of semi-insulating Si and an oxide 34 (such as, for example, SiO₂) that is grown or deposited on buffer layer 30, usually by Chemical Vapor Deposition (CVD). An optional release layer 36 of material which etches slower than the underlying oxide layer 34 is then formed over the oxide 34.

For example, a silicon oxy-nitride release layer, comprising a mixture of silicon nitride (S₃N₄) and silicon dioxide (SiO₂) may be a suitable choice. Such a layer etches more slowly in hydrofluoric acid than does SiO₂ alone. This etch rate can be controlled by adjusting the ratio of N and O in the silicon oxy-nitride (SiOₓN_{y}) compound.

A thin essentially single crystal layer 38 of silicon is then formed over the release layer 36. The oxide (or insulator) 34 is thus buried beneath the Si surface layer. For the case of ISE SOI structures, the top layer is essentially single-crystal recrystallized silicon, from which CMOS circuits can be fabricated.

Note: for the purposes of the present application, the term "essentially" single crystal means a film in which a majority of crystals show a common crystalline orientation and extend over a cross-sectional area in a plane of the film for at least 0.1 cm², and preferably, in the range of 0.5 - 1.0 cm², or more. The term also includes completely single crystal Si.

The use of a buried insulator provides devices having higher speeds than can be obtained in conventional bulk (Czochralski) material. Circuits containing in excess of 1.5 million CMOS transistors have been successfully fabricated in ISE material. An optional capping layer (not shown) also of silicon nitride may also be formed over layer 36 and removed when active devices are formed.

As shown in Fig. 4B, the film 38 is patterned to define active circuits, such as a TFT's in region 37 and a pixel electrode region at 39 for each display pixel. Note: For simplification, only one TFT 51 and one pixel electrode 62 is illustrated (Fig. 4H). It should be understood that an array of 1280 by 1024 such elements can in practice be formed on a single 6-inch wafer.

A plurality of arrays may be formed on a single six-inch wafer, which are then applied to the display as tiles and interconnected. Alternatively, the plurality of pixel matrices from one wafer can be separated and used in different displays. The plurality may comprise one large rectangular array surrounded by several smaller arrays (to be used in smaller displays). By mixing rectangular arrays of different areas, such an arrangement makes better use of the total available area on a round wafer.

An oxide layer 40 is then formed over the patterned regions including an insulator region 48 formed between the two regions 37, 39 of each pixel. The intrinsic crystallized material 38 is then implanted 44 (at Fig. 4C) with boron or other p-type dopants to provide a n-channel device (or alternatively, an n-type dopant for a p-channel device).

A polycrystalline silicon layer 42 is then deposited over the pixel and the layer 42 is then implanted 46, through a mask as seen in Fig. 4D, with an n-type dopant to lower the resistivity of the layer 42 to be used as the gate of the TFT. Next, the polysilicon 42 is patterned to form a gate 50, as seen in Fig. 4E, which is followed by a large implant 52 of boron to provide p+ source and drain regions 66, 64 for the TFT on either side of the gate electrode. As shown in Fig. 4F, an oxide 54 is formed over the transistor and openings 60, 56, 58 are formed through the oxide 54 to contact the source 66, the drain 64, and the gate 50. A patterned metallization 71 of aluminum, tungsten or other suitable metal is used to connect the exposed pixel electrode 62 to the source 66 (or drain), and to connect the gate and drain to other circuit panel components.

The devices have now been processed and the circuits may now be tested and repaired, as required, before further processing occurs.

The next step in the process is to transfer the silicon pixel circuit film to a common module, either directly, or by a double transfer from substrate to carrier and then to the common module. A double transfer approach is illustrated in Figs. 4H-4L. To separate a circuit tile from the buffer 30 and substrate 37, a first opening 70 (in Fig. 4H) is etched in an exposed region of release layer 36 that occurs between tiles. Oxide layer 34 etches more rapidly in HF than nitride layer 36, thus a larger portion of layer 34 is removed to form cavity 72. A portion of layer 36 thus extends over the cavity 72.

In Fig. 4I, a support post 76 of oxide is formed to fill cavity 72 and opening 70, which extends over a portion of layer 36. Openings or via holes 74 are then provided through layer 36 such that an etchant can be introduced through holes 74, or through openings 78 etched beneath the release layer 36, to remove layer 34 (See Fig. 4J). The remaining release layer 36 and the circuitry supported thereon is now held in place relative to substrate 32 and buffer 30 with support posts 76.

Next, an epoxy 84 that can be cured with ultraviolet light is used to attach an optically transmissive superstrate 80 to the circuitry, and layer 36. The buffer 30 and substrate 32 is then patterned and selectively exposed to light such that regions of epoxy 84' about the posts 76 remain uncured while the remaining epoxy 84' is cured (See Fig. 4K). The buffer 30 and substrate 32 and posts 76 are removed by cleavage of the oxide post and dissolution of the uncured 84 epoxy to provide the thin film tile structure 141, shown in Fig. 4L mounted on carrier 80.

To form the final display panel, the edges of the carrier 80 are trimmed to coincide with the tile borders. The nitride release layer 36 is removed by etching.

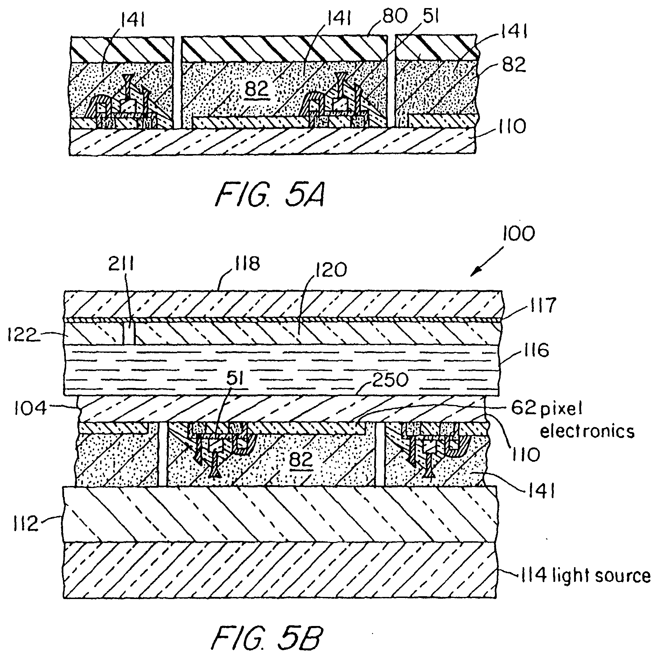

As shown in Fig. 5A, a plurality of tile structures 141 are then sequentially registered with one another and adhered to a common module body 110 using a suitable adhesive (not shown). Common module body 110 is preferably patterned with interconnect metallization on the surface facing the tile structure 141 for interconnecting individual tile circuitry with each other. Next, insulation and alignment layers, spacers, a sealing border and bonding pads for connections (not shown) are bonded onto the periphery of the common module body 110. A screen printing process can be used to prepare the border. As shown in Fig. 5B, a plate 117 containing the color filters 120 and the counterelectrode (not shown) is bonded to the periphery thin film circuit tiles 141 with the sealing border after insertion of spacers (not shown). The display is filled with the selected liquid crystal material 116 via a small filling hole or holes extending through the border. This filling hole is then sealed with a resin or epoxy. First and second polarizer films 118, 112 or layers are then bonded to both sides and connectors (not shown) are added. Finally, a white light source 114, or other suitable light source, is bonded to polarizer 112.

Pixel electrodes 62 are laterally spaced from each other. Each pixel has a transistor 51 and a color filter 120 or 122 associated therewith. A bonding element or adhesive 82 and optically transmissive superstrate 110, such as glass or plastic completes the structure. Body 110 is preferably a low temperature glass that can have a thickness preferably of about 200 to 1000 microns.

In an alternative CLEFT process, thin single-crystal films, are grown by chemical vapor deposition (CVD), and separated from a reusable homoepitaxial substrate.

The films removed from the substrate by CLEFT are "essentially" single-crystal, of low defect density, are only a few microns thick, and consequently, circuit panels formed by this process have little weight and good light transmission characteristics.

The CLEFT process, illustrated in U.S.-A-4,727,047, involves the following steps: growth of the desired thin film over a release layer (a plane of weakness), formation of metallization and other coatings, formation of a bond between the film and a second substrate, such as glass (or superstrate), and separation along the built-in-plane of weakness by cleaving. The substrate is then available for reuse.

The CLEFT process is used to form sheets of essentially single crystal material using lateral epitaxial growth to form a continuous film on top of a release layer. For silicon, the lateral epitaxy is accomplished either by selective CVD or, preferably, the ISE process or other recrystallization procedures. Alternatively, other standard deposition techniques can be used to form the necessary thin film of essentially single crystal material.

One of the necessary properties of the material that forms the release layer is the lack of adhesion between the layer and the semiconductor film. When a weak plane has been created by the release layer, the film can be cleaved from the substrate without any degradation. As noted in connection with Figs. 4A-4C, the release layers can comprise multi-layer films of Si₃N₄ and SiO₂. Such an approach permits the SiO₂ to be used to passivate the back of the CMOS logic. (The Si₃N₄ is the layer that is dissolved to produce the plane of weakness.) In the CLEFT approach, the circuits are first bonded to the glass, or other transfer substrate, and then separated, resulting in simpler handling as compared to, for example, UV-cured tape.

The plane of weakness is key to obtaining uniform cleaving between the circuits and the substrate. This plane may be formed by creating a pattern of carbon on the surface of the wafer so that only a small fraction of the underlying semiconductor surface is exposed. These exposed portions are used as nucleation cites for the epitaxial film. If the growth conditions are properly chosen, the film will grow laterally faster than vertically, leading to laterial overgrowth of the single crystal film. Within lim of vertical growth, the film becomes continuous and of high quality. However, the carbon layer is weak and, combined with the small fraction of exposed semiconductor areas where the film is strongly attached to the substrate, creates a plane of weakness. This plane can be used reliabily and reproducibly to separate the film from the substrate. The substrate may be reused. These processes have been used to transfer a wide range of GaAs and Si circuits to alternative substrates such as glass, ceramic, and other materials, without harm to the active circuitry.

In the ISE process, the oxide film is strongly attached to the substrate and to the top Si film which will contain the circuits. For this reason, it is necessary to reduce the strength of the bond chemically. This requires use of a release layer that is preferentially dissolved with an etchant without complete separation to form a plane of weakness in the release layer. The films can then be separated mechanically after the glass is bonded to the circuits and electrodes.

Mechanical separation may be accomplished by bonding the upper surface of the Si film to a superstrate, such as glass, using a transparent epoxy. The film and glass are then bonded with wax to glass plates about 5 mm thick that serve as cleaving supports. A metal wedge is inserted between the two glass plates to force the surfaces apart. Since the mask has low adhesion to the substrate, the film is cleaved from the substrate but remains mounted on the glass. The substrate can then be used for another cycle of the CLEFT process, and the device processing may then be completed on the back surface of the film. Note that since the device remains attached to a superstrate, the back side can be subjected to standard wafer processing, including photolithography.

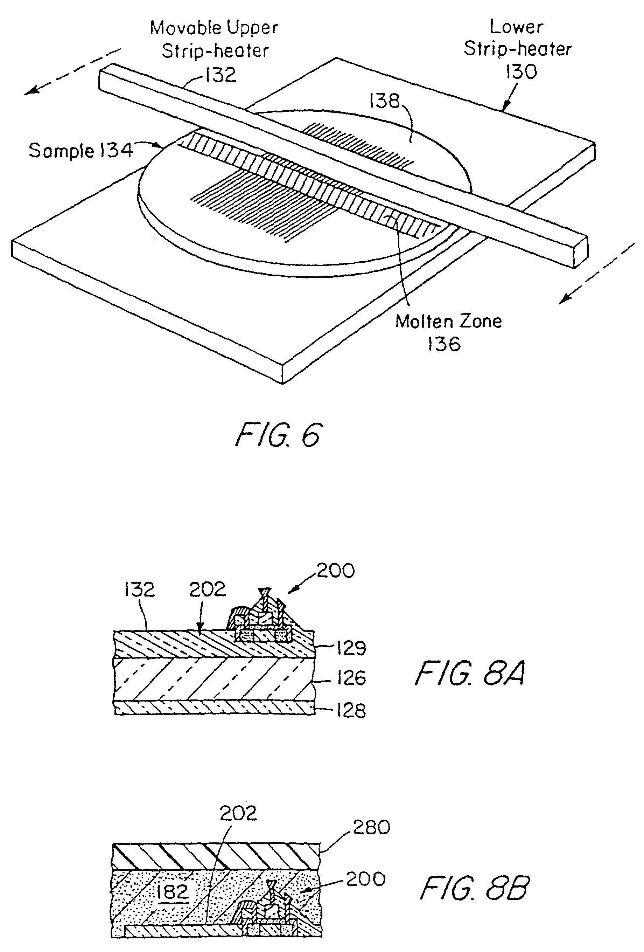

One example not forming part of the invention utilizes a recrystallization system, shown schematically in Fig. 6 to form the essentially single crystal Si thin film. A sample wafer 134 is formed of poly Si, formed on SiO₂, formed on an Si wafer. A capping layer 138 is formed over the poly Si. The wafer temperature is then elevated to near the melting point by a lower heater 130. An upper wire or graphite strip heater 132 is then scanned across the top of the sample 134 to cause a moving melt zone 136 to recrystallize or further crystallize the polycrystalline silicon. The lateral epitaxy is seeded from small openings formed through the lower oxide. The resultant single crystal film has the orientation of the substrate.

A number of unique devices and circuits have been formed using the above processing techniques. These techniques have been used to transfer CMOS active matrix LCD circuitry from ISE wafers to glass, and have yielded excellent displays with single crystal Si active matrix circuits. Silicon circuitry has been transferred to glass and shows no important changes in transistor charcteristics after transfer. The technique has also been proved with III-V compound semiconductor circuits. For example, GaAs and AlGaAs monolithic series-connected photovoltaic energy converters have been made for power down a fiber application that yield exceptional performance. Also, two-dimensional multiplexed AlGaAs LED arrays (with over 32K pixels) have been made by transfer and two-sided processing and exhibit extremely high LED density as well as performance. The development of this broad range of Si and III-V circuits indicates the general applicability of the transfer process to a wide range of devices and circuits.

### III. Alternate Adhesion and Transfer Processes

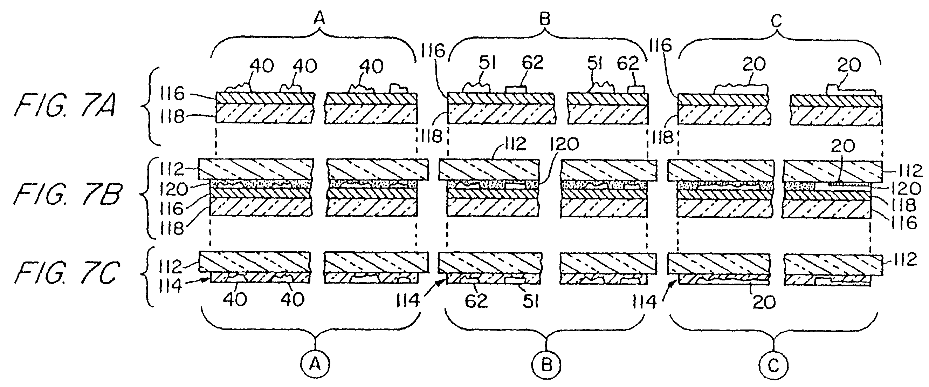

Figs. 7A-7D illustrate an alternate preferred double transfer process for adhering and transferring tiles of circuits of thin films of silicon to a common module body. The starting structure is a silicon wafer 118 upon which an oxide layer 116 and a thin film of poly-Si, Á-Si or x-Si 114 is formed using any of the previously described processes such as ISE or CLEFT. A plurality of circuits, such as pixel electrodes, TFT's, Si drivers and Si logic circuits, are then formed in the thin film. Fig. 7A shows three such wafers, I, II, III. In wafer I, logic circuits 40 are formed. In wafer II, pixel electrodes 62 and TFT's 51 are formed. In wafer III, driver circuits 20 are formed. A wafer, or individual tiles diced from the wafer, is attached to a superstrate transfer body 112, such as glass or other transparent insulator, using an adhesive 120. Preferably the adhesive is comprised of commercially available epoxies.

The wafer, or tile, is then cleaned and the native oxide 118 is etched off the back surface. Depending on the thickness of the wafer, it may take up to 5 hours to etch the Si 118 and oxide 116 layers. The solution etches silicon very rapidly, i.e. 2 to 3 microns/min., and uniformly if the wafers are held horizontally in the solution with the etching surface face up. The acid has a very low etch rate on oxide, so that as the substrate is etched away and the buried oxide is exposed, the etching rate goes down. The observer can monitor the process and to stop the etch in the buried oxide layer 116' without punching through to the thin silicon layer 114 above it. Wafers up to 25 mils (1 mil = 25,4 x 10⁻⁶ m) thick and oxides as thin as 4000Å (1 Å = 10⁻¹⁰ m) have been successfully etched using this process. An alternative etchant is hydrazine, which has a much higher etch rate selectivity or ethylene diamine pyrocatacol (EDP).

When the silicon is completely gone, the vigorous bubbling, which is characteristic of silicon etching abruptly stops, signalling that the etching is complete.

The thin films 114 transferred to the respective glass superstrates 112 are now rinsed and dried. If not already provided with circuits 40, 51, 62 or 20, the films 114 can be backside circuit processed, if desired.

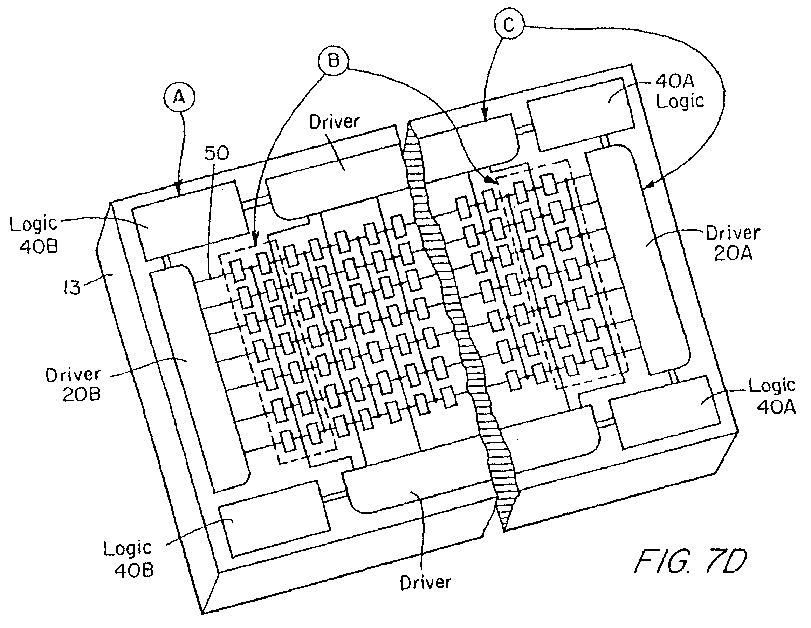

After all the necessary circuits are formed, as above, on transfer bodies 112, they may now be diced and tiled onto a common module body 13 (Fig. 7D) to perform a combined function, such as an AMLCD.

The logic circuits 40 of transfer body 118 in col. A, Fig. 7C, are transferred to the border of module body 13, while the driver circuits 20 from the transfer body 118 in col. C, Fig. 7C, are disposed on the border between the logic circuits 40A and 40B.

Tiles of pixel electrodes 62 and TFT's 51 are formed by dicing or etching and are registered with respect to each other and pre-formed wiring 50 on module body 13, as shown.

After all the circuits are registered and adhered to the module body, the transfer body 118 and the epoxy 120 is removed using a suitable etchant, such as HF for the case of a glass transfer body.

Interconnection of circuits is achieved during registration or by direct laser writing where necessary. Also, if desired, the film can be transferred to another substrate and the first glass superstrate and adhesive can be etched off, allowing access to the front side of the wafer for further circuit processing.

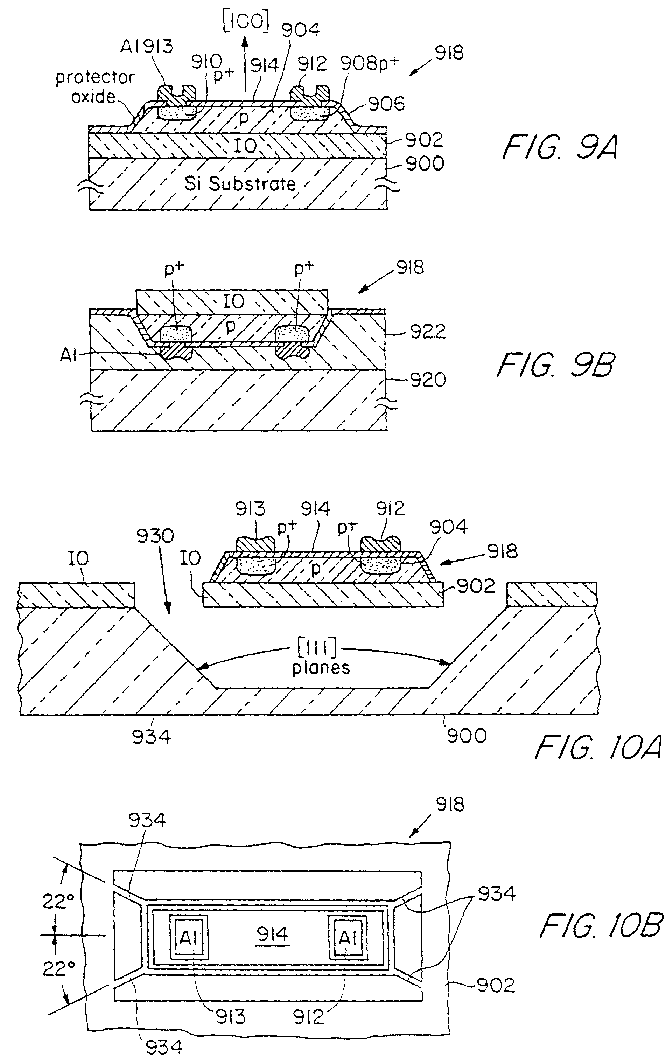

Figs. 8A and 8B illustrate an alternative one-step silicon thin film transfer process in which GeSi is used as an intermediate etch stop layer. In this process, Si buffer layer 126 is formed on an x-Si substrate 128 followed by a thin GeSi layer 129 and a thin Á-Si, poly-Si, or x-Si device or circuit layer 132; using well-known CVD or MBE growth systems.

The layer 132 is then IC processed in the manner previously described in connection with Figs. 4E-H, to form circuits, such as TFT's 200 and pixel electrodes 202 (Fig. 8A). Next, the processed wafers, or tiles from the wafer, are mounted on a common module glass (or other) support 280 using an epoxy adhesive of the type previously mentioned in connection with Figs. 7A-7B. The epoxy fills in the voids formed by the previous processing and adheres the front face to the superstrate 280.

Next, the original Si substrate 128 and Si buffer 126 are removed by etching, which does not affect the GeSi layer 129 (Figs. 8B). Finally, the GeSi layer 124 is removed by brief submersion in a suitable etch.

### IV. Pressure Sensor Example

Figs. 9A-9B illustrate an alternate example not forming part of the invention related to isolating and transferring circuits. In a representative example, a method of fabricating pressure sensing transducers on a glass substrate is shown in Figs. 9A-9B and described hereinafter. The transducer circuit operates by sensing a change in the resistance of the p-region 904 in response to pressure applied to the circuit. This resistance change may be sensed by an ohmmeter coupled across contacts 912 and 912 and calibrated and converted into a pressure sensor to serve as a strain gauge. The starting structure is shown in Fig. 9A. An SOI wafer is provided which consists of an Si substrate 900 beneath a buried oxide layer 902, upon which is formed a single, or nearly single, crystal Si layer 904. A blanket implant of boron ions is made to make the Si layer a p-type conductor. A thin (1000Å) blanket protective/mask layer of oxide (SiO₂) (not shown) is then formed over the structure. (Note Fig. 9A shows the structure after processing). Single, or nearly single, islands of x-Si are then formed by applying photo-resist over the oxide structure and etching the oxide and silicon 904 between islands to align the edges of the islands parallel to the [110] plane. Photo resist is applied again and contact openings formed to contact regions 910 and 908, which are then implanted with a high dose of boron ions to form P⁺ type conductivity regions. A protective oxide layer 906 is then formed over the island. Aluminum contact pads, 912 and 913 to the contacts 908, 910 are formed in openings provided through oxide 906. The pressure transducer circuit of Fig. 9A is now ready for transfer to a temporary glass substrate.

After the circuit 918 is formed, the circuit is transferred to a temporary substrate 920 using a removable epoxy 922. The silicon substrate 900 is etched away. Then using a photoresist and mask the initial oxide layer 902 is etched around the periphery of the circuit 918 leaving the circuit free to be inverted and transferred to the glass substrate 920 and releasibly bonded thereto using the removable epoxy 922 from which it can be transferred and bonded to a module for general sensing, including temperature, pressure, acceleration, and so forth, all under microprocessor supervision, to make a high speed process controller.

Figs. 10A and 10B illustrate an alternate transfer process in which the initial oxide 902 is etched about the periphery of each circuit 918 using a conventional photo-resist and mask technique. The Si substrate 900 is also etched locally which preferentially etches Si to reveal the [111] plane. A nitride layer may be added such that the etchant does not etch the aluminum. Etching of the Si substrate with hydrazine undercuts the circuits 918 forming a cavity 930 under the circuits and leaving a bridge structure 934 between circuits 918 and the substrate.

When it is desired to remove one or more circuits 918, a vacuum wand may be used to seize one or more circuits and break the bridge to remove the circuits which may then be transferred along with other circuits to a common module substrate and aligned and interconnected with other circuitry to perform an overall function as previously described. Alternatively, other techniques such as laser ablation can be used for removing the circuits from the substrate.

Fig. 10B is a top-plan view of Fig. 10A before substrate 900 is etched where the bridges 934 are shown. The bridges make an angle of about 22 relative to the long symmetry axis of the circuit 918.

### V. Three-Dimensional Circuitry

### A. 3-D Circuit Architecture

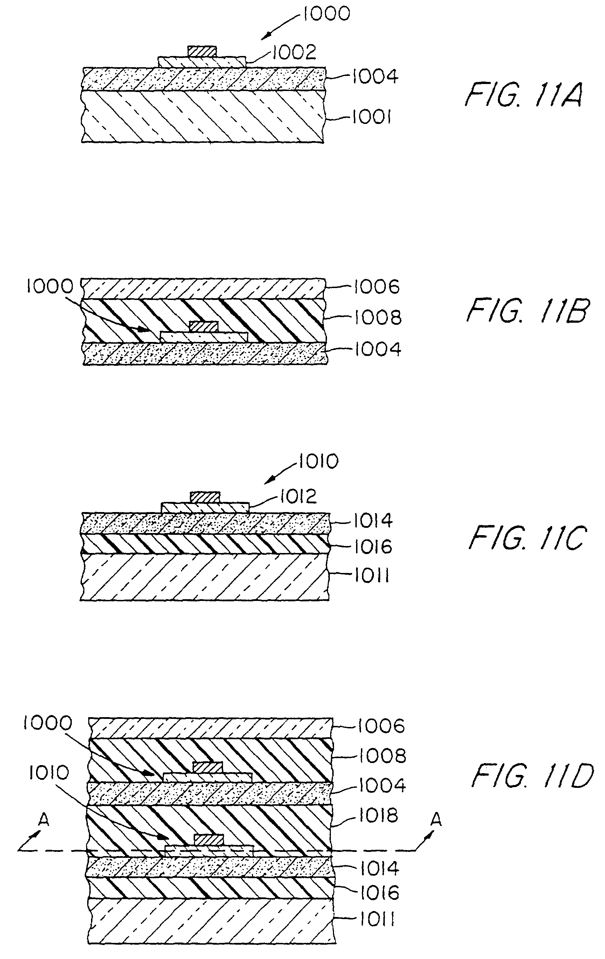

Other examples not forming part of the present invention relate to the formation of three-dimensional circuits. In forming a two-layer three-dimensional circuit, a first circuit 1000 (Fig. 11A) formed in a silicon layer 1002 on an oxide layer 1004 on an Si substrate 1001 is transferred onto a glass superstrate 1006 as shown in Fig. 11B. More specifically, the single-transferred circuit 1000 is transferred to a glass superstrate, coated with amorphous silicon, by any of the aforementioned transfer methods and bonded to the glass with an adhesive or epoxy 1008. Referring to Fig. 11C, a second circuit 1010 is double-transferred to a glass or diamond substrate 1011. The circuit 1010 is preferably formed in a layer of silicon 1012 on an oxide layer 1014, and is bonded to the substrate by a layer of adhesive or epoxy 1016.

Referring to Fig. 11D, a three-dimensional device is formed by bonding the single-transferred circuit 1000 (Fig. 11B) on top of the double-transferred circuit 1010 (Fig. 11C) using thin, uniform adhesive 1018. Since the circuits can be observed through the glass substrate 1011, they can be aligned using a microscope or a contact or proximity aligner as routinely done in photolithography where a mask is aligned on top of a silicon circuit in process or by other appropriate micropositioning tools or techniques.

After bonding, the superstrate 1006 is removed as in a double-transfer process and the adhesive 1008 is removed in oxygen plasma. This leaves the front surface of the top circuit 1000 exposed. The bottom circuit 1010 is buried beneath the adhesive layer 1018. In order to make connections between the layers of circuits, openings or via holes (not shown) are defined by appropriate etchants in order to expose contact areas on the two circuit layers. All of the oxide is etched in buffered HF using photoresist as a mask while the adhesive can be etched in oxygen plasma or by reactive ion etching (RIE) using the previously etched oxide as a mask. Once these via holes have been opened in the bonding layer, they can be filled with metal in order to make the contact from layer to layer. The layer to layer interconnections are explained in detail below. The adhesive layer between the superposed circuits must be kept very thin, 1-25 microns, preferably 5 microns thick to facilitate layer to layer contacting. The process can be repeated to add additional layers to the device.

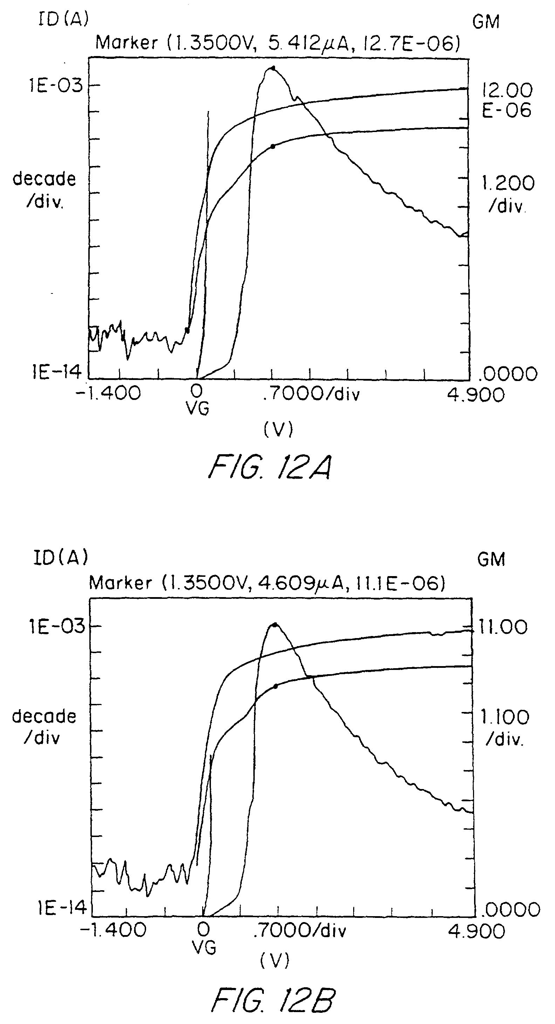

The performance characteristics of each circuit in a three-dimensional structure are related to the thermal conductivity of the medium in which the circuit is disposed. Figs. 12A-12B show performance curves of a lower MOSFET circuit of a three-dimensional device (such as in Fig. 11D) and the corresponding curves for a similar device after double-transfer and before three-dimensional mounting (such as in Fig. 11C). The graphs of Figs. 12A-12B show that the transconductance and the drive current are higher when the circuit is buried under epoxy (Fig. 11D) than when it is exposed to ambient air (Fig. 11C). This effect can be explained by a higher thermal conductivity of the epoxy with respect to air which results in a reduced heating effect for the circuit buried in epoxy (Fig. 11D). It is noted that carrier mobility decreases as the temperature of the circuit increases and that performance is directly related to carrier mobility. Thus, surrounding circuits in highly conductive epoxies provide lower device temperatures leading to improved performance characteristics. These epoxies can be filled with particles of thermally materials such as diamond aluminum nitride, silicon carbide, and other conductive compounds. There are many available thermally conductive/electrically insulating epoxies.

An advantage of this approach is the capability to integrate heat sink layers within the stack. Heat dissipation is an important problem in 3D architectures, and a capability for insertion of heat conducting layers between active electronic layers is advantageous. These layers can have much higher thermal conductivity than can be attained in Si or other semiconductors that may be envisioned for heat transfer.

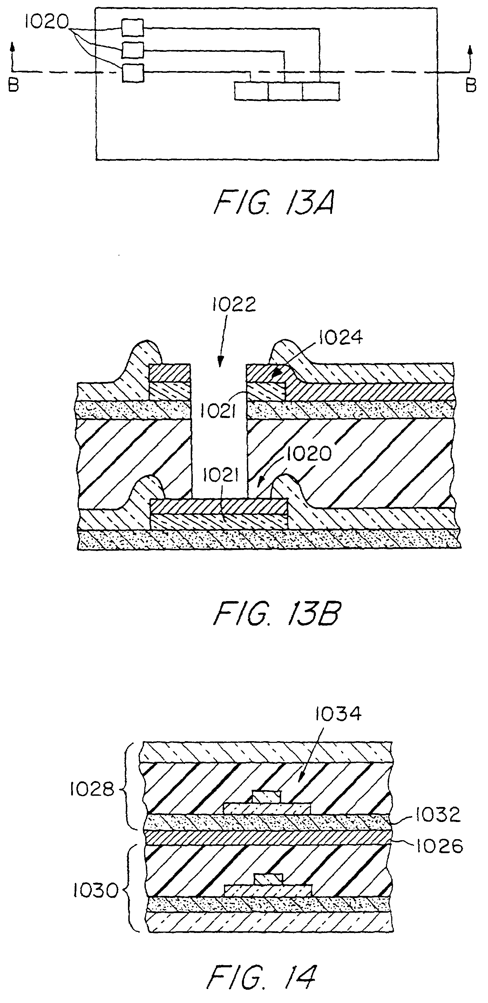

One significant aspect in the formation of three-dimensional circuits involves interconnecting the layered devices. It is noted that in such circuits, the epoxy disposed between the device layers may be spun to obtain a thickness of a few microns. Alternatively, other known techniques can be employed to obtain a thin, uniform layer of epoxy. Fig. 13A is a sectional view of Fig. 11D taken along the line A-A and shows the lower contact area 1020 formed via metalization in the plane of the silicon layer 1012 for providing electrical connection to the circuit 1010 (Fig. 11D). Similarly, upper contact areas (not shown) are formed directly above the lower areas in the plane of the silicon layer 1002 and are electrically connected to the upper circuit 1000 (Fig. 11D). Referring to Fig. 13B, the upper and lower areas (1024, 1020) employ an optional poly-Si layer for strengthening the areas for contacts. Via holes 1022 are formed through the upper contact areas 1024 to gain access to the lower contact areas 1020. The etching to form the via holes with high aspect ratio is performed by an RIE technique. Electrical contact between the upper and lower devices is made by filling the via holes 1022 with an electrically conductive material such as tungsten or aluminum.

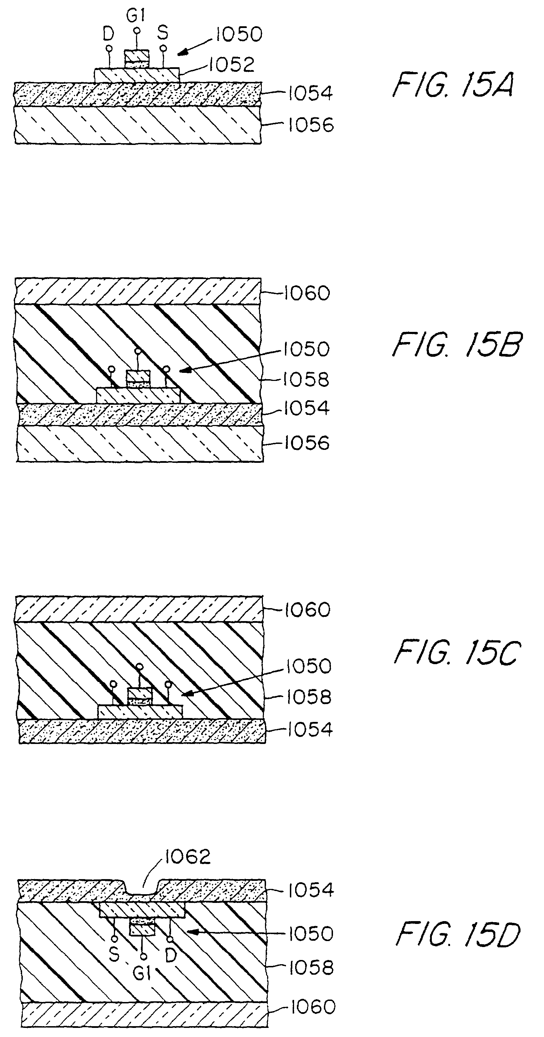

Another significant aspect of three-dimensional circuits involves shielding device layers to avoid undesirable electrical or magnetic interference between devices. Referring to Fig. 14, ground planes 1026 are positioned between device layers 1028 and 1030 to prevent electrical interference. These conductive ground planes 1026 can be made with a metal or by ITO deposition on the surface of the oxide layer 1032 opposite the device 1034. Alternatively, the ground planes can be formed with an electrically conductive epoxy or with a highly doped silicon layer taking the place of a device layer in the stacked structure.

A benefit of using transfer technology to form a multilayer stack is attained if superior radiation resistance and low weight are desired. The removal of the substrate decreases the probability of single event upset and reduces the weight, but also important is the potential for utilization of high-Z radiation shielding material at the outside surfaces of the device. This shielding can protect many layers of stacked circuits with much less increase in weight than is needed for a 2D geometry. Thus, there are important benefits for space applications in which radiation resistance and weight are important factors.

A further benefit of the proposed technology is that the multi-layer circuit may be made tamper resistant. This benefit is particularly important for the prevention of reverse engineering for proprietary circuits. In a 2D circuit, the die can be removed from the package and examined by microanalytic techiques. However, a 3D circuit can be formed in such a way that separation of the layers is not only difficult, but also highly destructive, so that separation of the layers would not yield meaningful patterns or useful reverse-engineered data.

Speed advantages can be attained by using a 3D approach. For example, in a memory comprising stacked 2D circuits access time can be reduced because the transit distance for address signals is shorter than a comparable tilted planar 2D memory. As another example by using a 3D addressing scheme for a true 3D architecture, access time may be reduced to an even lower level.

Transfer of circuits into a 3D architecture can also permit the integration of high speed GaAs analog signal processing circuitry (MMICs) with silicon circuits. This permits the integration of microwave circuits with high speed highly dense Si circuits. Such versatility may not be easy to obtain with other approaches. Transfer is also applicable to digital GaAs circuitry.

Finally, the transfer approach, makes possible the use of optoelectronic circuits and devices. These circuits can be used for optical computing, optical I/O, or optical interconnects between circuit planes in the 3D structure. Other uses of optoelectronic devices or circuits can be integrated relatively easily if a need for these devices arises.

This technology makes possible the vertical integration of memory layers, leading to new circuit architectures. Specific advantages include the following:
- Extremely rapid access time,
- New memory architectures that are more compatible with neural network memory models, and
- Compatibility with optical processing and optical addressing of the memory.

These advantages are a result of the capability not only to integrate connections between layers, but also to interpose optoelectronic devices and circuits within the layers. Thus, the proposed technology may form the basic building block of an entirely new type of circuit architecture.

### B. 3-D Device Formation

In accordance with the present invention, a fabrication process comprising single and double transfer steps and a backside processing step can be employed to provide various 3-D devices. The fabrication process includes the formation of circuits in a Si film of an SOI structure, adhering the circuits to a superstrate and removal of the substrate. At this point, the silicon circuits have been single-transferred and the backside of the silicon circuit layer is exposed. Backside processing can be performed so long as the processing is compatible with the selected adhesive. After backside processing is performed, the silicon circuit layer is transferred to a glass substrate (double-transfer).

In one preferred example not forming part of the invention, a double gate MOSFET can be formed in accordance with the above-described fabrication process. First, a standard MOSFET device 1050 having a drain (D), a gate (G1) and a source (S) (Fig. 15A) is formed by an suitable method such as described previously herein. The next step in the process is to transfer the device film 1052 from its substrate 1056 to a superstrate for backside processing. A single transfer approach is shown in Figs. 15B-15D. Referring to Fig. 15B, an epoxy 1058 is used to attach an optically transmissive superstrate 1060. In a preferred example not forming part of the invention, a glass superstrate coated with Á-Si is employed with a two-part epoxy. Once the front surface of the film 1052 has been bonded to the superstrate 1060, the substrate 1056 is etched. As shown in Fig. 15C, the etchant rapidly removes the silicon substrate 1056 with the oxide layer 1054 serving as an etch stop. The etch rate selectivity of 200:1 for silicon versus thermal silicon dioxide allows the use of very thin oxide layers leaving the device 1050 protected from the etchant.

After single transfer, using an opposite polarity gate mask (not shown) the oxide layer 1054 is thinned down to a few hundred angstroms (∼500Å) along the channel region 1062 (Fig. 15D). An alternative method of providing a thin oxide layer adjacent the backside of the MOSFET device 1050 is illustrated in Fig. 15E. Once again using a mask (not shown), the oxide layer along the channel region 1062 is etched away to expose the backside of the device 1050. Next, a thin oxide layer 1063 (∼500Å) can be deposited in the region 1062.

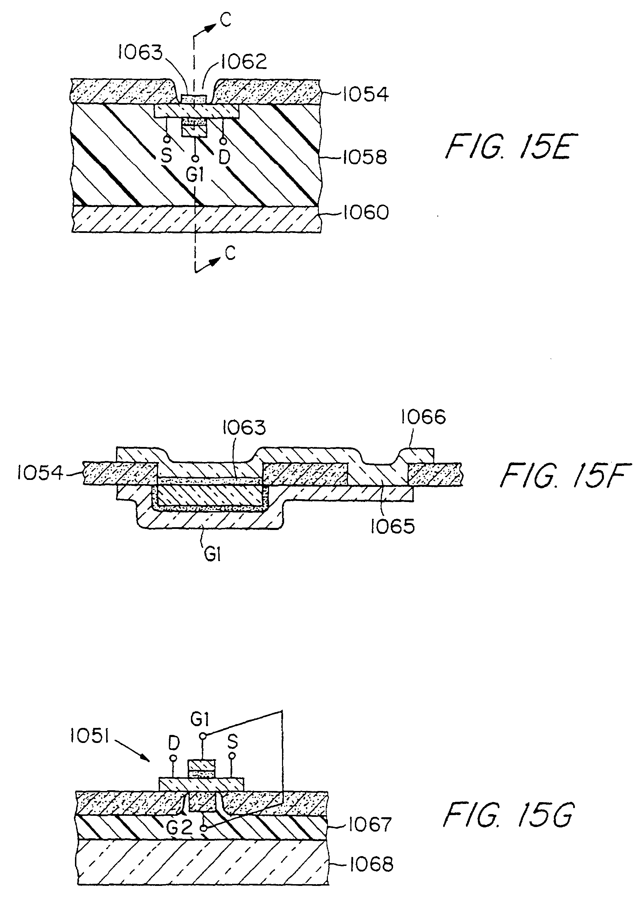

A second gate (G2) is then formed over the thin oxide layer 1063 and electrically connected to the first gate (G1) as follows. Referring to Fig. 15F, which is a cross-sectional view of the structure shown Fig. 15E a contact hole 1065 can be opened through the thinned oxide, and a gate material (1066) can be deposited and etched to form a second gate (G2) 1064 which is electrically connected to the first gate (G1). This dual gate configuration serves to practically double the drive current for the MOSFET 1051 since the device has two channels. Referring to Fig. 15G, the dual gate MOSFET 1051 may be transferred again and bonded with epoxy 1067 to a permanent substrate 1068 such as glass.

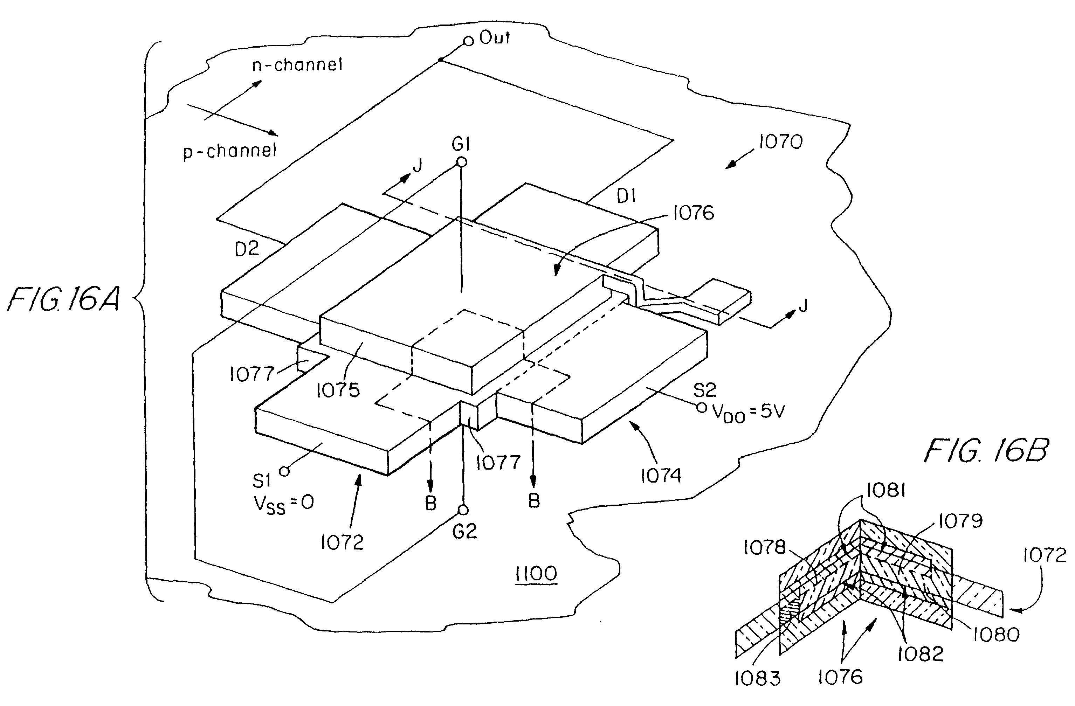

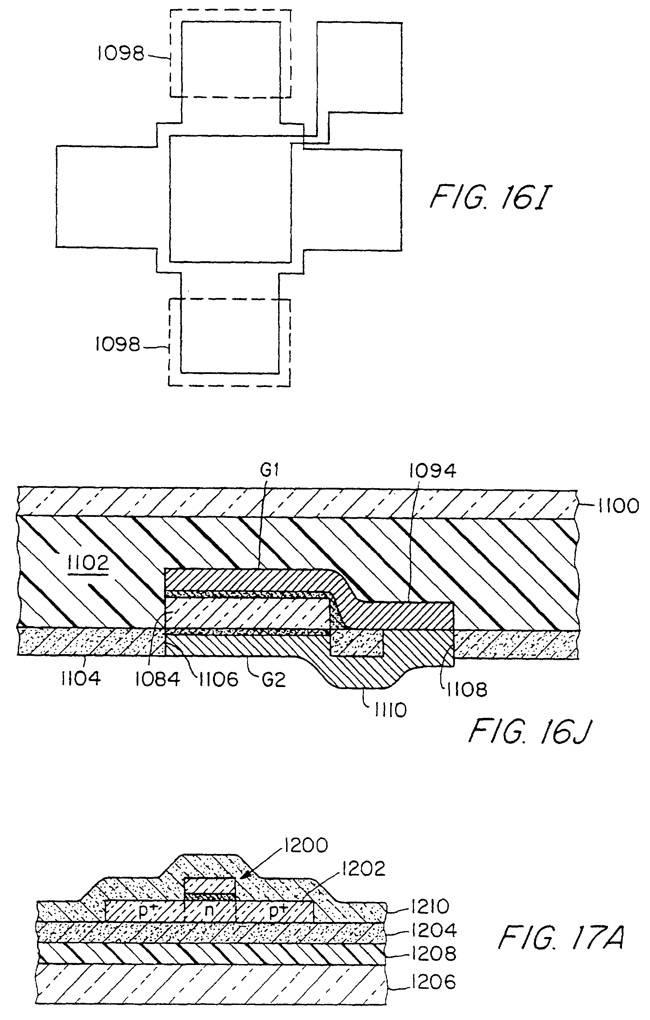

In another preferred example not forming part of the invention, a 3-D double-gate MOSFET inverter 1070 can be fabricated such that the n-channel and p-channel MOSFETS share the same body with their channels disposed on opposite sides thereof. The fabrication sequence for providing a double-gate inverter is shown in Figs. 16A-16J. Referring to Fig. 16A, the device 1070 includes an n-channel MOSFET 1072 with a gate (G1), source (S1) and drain (D1) and a p-channel MOSFET 1074 with a gate (G2), source (S2) and a drain (D2). Referring to Fig. 16B, the shared region 1076 includes the n-channel 1078 and the p-channel 1080 which are disposed on opposite sides of the region. More specifically, the channel for the n-channel MOSFET is disposed along the top interface 1081 of the shared region and the channel for the p-channel MOSFET is disposed along the bottom interface 1082 of the shared region.

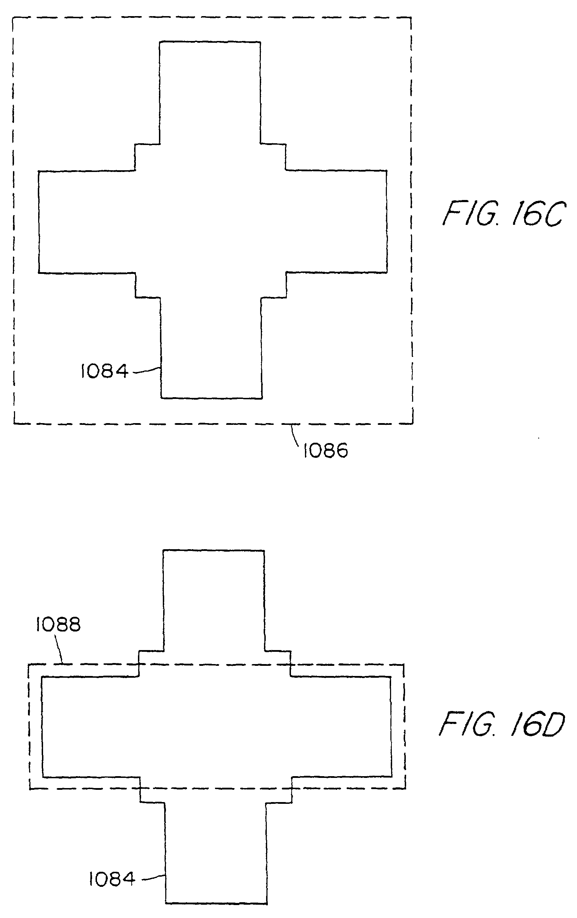

A series of plan views illustrating the processing steps employed for fabricating a double gate MOSFET inverter are shown in Figs. 16C-16J. Fig. 16C illustrates the channel doping for the p-channel MOSFET. A photoresist and a mask are positioned over the patterned silicon island 1084 and phosphorous (or other n-type dopants) is implanted into the area 1086 with a projected range (Rₚ) near the bottom interface 1082 (Fig. 16B). The implant is such that the phosphorous concentration at the bottom interface is about 10¹⁶ cm⁻³. Fig. 16D illustrates the channel doping for the n-channel MOSFET. Using a photoresist and mask, boron (or other p-type dopants) is implanted in the area 1088 with an Rₚ near the top interface 1081 (Fig. 16B). The implant preferably produces a boron concentration at the top interface of about 4 x 10¹⁶ cm⁻³.

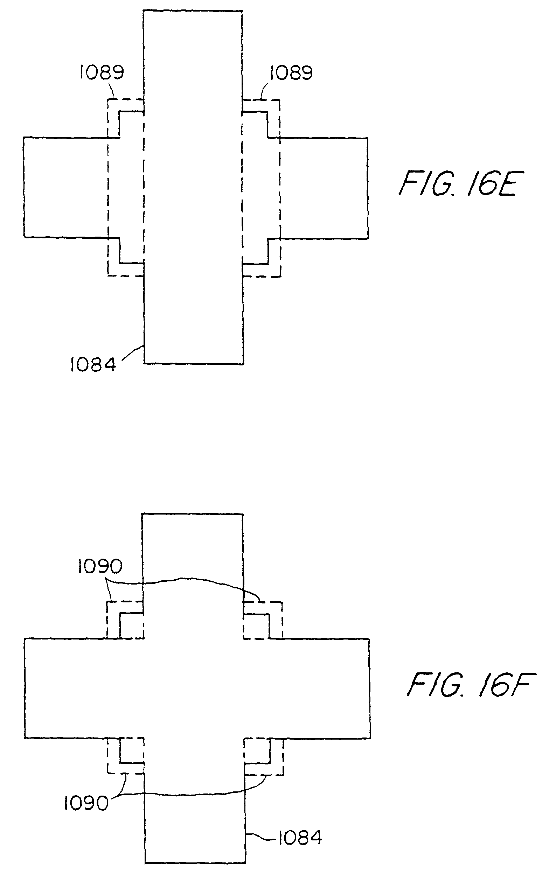

Fig. 16E illustrates the formation of the channel stop 1083 (Fig. 16B) for the n-channel MOSFET. A photoresist and mask are positioned over the silicon island such that boron is implanted into the regions 1089 with an Rₚ in the in the middle of silicon. This implant is such that the average boron concentration in the middle of the silicon is about 4 x 10¹⁶ cm⁻³. Fig. 16F illustrates an edge implant for the n-channel MOSFET. To avoid the effect of sidewall parasitic transistors, the corner regions 1077 (Fig. 16A) extend beyond the gate material preventing the gate from contacting the sidewall of the silicon island to form a sidewall transistor. Further, these corner regions are heavily doped to minimize sidewall transistor effects on the double-gate inverter. Using a photoresist and mask, boron (or other p-type dopant) is implanted into the areas 1090 with an Rₚ near the top interface. The implant preferably produces a boron concentration at the top interface of about 5 x 10¹⁷ cm⁻³.

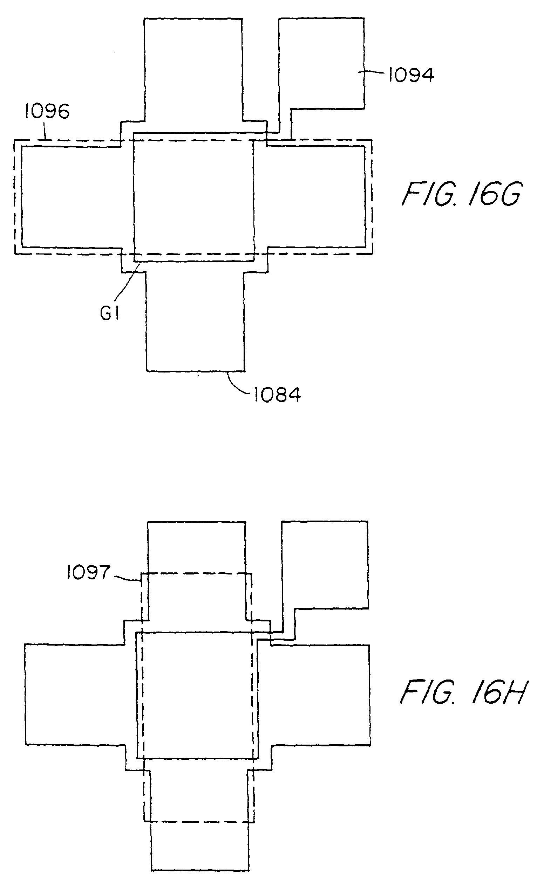

Referring to Fig. 16G, the gate (G1) and the contact area 1094 are then formed for n-channel MOSFET. Next, the source/drain doping is performed for the n-channel device. Using a photoresist and mask, arsenic (or other n-type dopants) is implanted, self-aligned with the gate (G1), into the area 1096 with an Rₚ near the top interface and an arsenic concentration of about 10²⁰ cm⁻³. Fig. 16H illustrates the formation of the channel stop 1079 (Fig. 16B) for the p-channel MOSFET. Using a photoresist and mask, phosphorus (or other n-type dopants) is implanted, self-aligned with the gate (G1), into the area 1097 with an Rₚ near the top interface 1081 (Fig. 16B) and a phosphorus concentration of about 8 x 10¹⁶ cm⁻³. Next, the source/drain doping is performed for the p-channel MOSFET. Again using a photoresist and mask, boron is implanted into the areas 1098 with a Rₚ in the middle of the silicon and an average boron concentration of 10²⁰ cm⁻³.

Next, the gate (G2) is formed for the p-channel MOSFET and electrically connected to the gate (G1). Referring to Fig. 16J (which is a sectional view of Fig. 16A taken along the line J-J), the double-gate MOSFET is single transferred to a temporary superstrate 1100 and attached to the superstrate by an adhesive or epoxy 1102. Then, the oxide layer 1104 upon which the device is disposed is selectively etched using a photoresist and a mask to open two areas 1106 and 1108. Next, the gate (G2) is formed in the area 1106 by metalization as well as the contact path 1110 to the contact area 1094. After metallization, the two gates are electrically connected.

In a preferred embodiment of the invention, a three-dimensional inverter, is formed with a pair of MOSFETs which are vertically stacked as shown in Fig. 17D. The fabrication process for the three-dimensional inverter is shown in Figs. 17A-17D. Referring to Fig. 17A, an n-channel device 1200 is formed in single crystal silicon 1202 on an oxide 1204 over a substrate (not shown). After a double transfer, the device 1200 is attached with an adhesive or epoxy 1208 to a substrate (1206). A passivation oxide layer 1210 is deposited over the device 120.

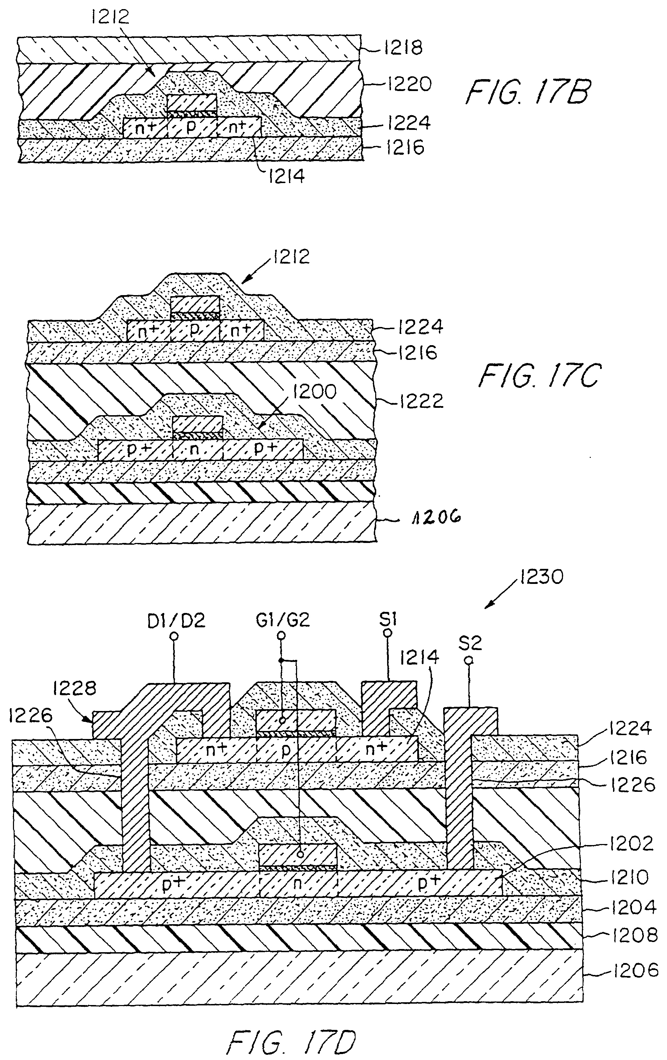

Referring to Fig. 17B, a p-channel device 1212 is separately fabricated in single crystal silicon 1214 on an oxide 1216 on a substrate (not shown). An oxide layer 1224 is deposited over the p-channel device 1212 for passivation, and single transfer is performed such that the device is attached to a superstrate 1218 by an adhesive 1220. The p-channel device 1212 is then attached to the n-channel device by an adhesive 1222 forming a stacked structure (Fig. 17C).

Next, an oxide layer 1224 is deposited over the p-channel device 1212 for passivation. Referring to Fig. 17D, vias 1226 are then formed to access the gate, source and drain regions of the upper device 1212 and the buried device 1200. A metal layer 1228 is deposited and patterned to form electrical interconnects, for the stacked inverter structure 1230. It is noted that the interconnection of the respective gates is made in a plane parallel to the figure such that the vias are not shown.

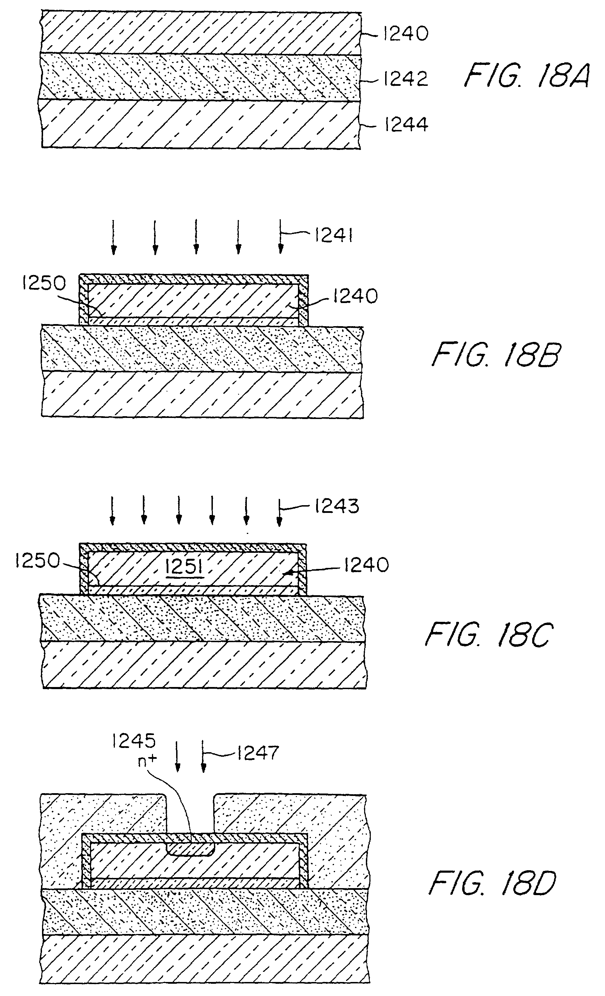

In yet another preferred example not forming part of the invention, a vertical bipolar transistor is fabricated in accordance with the principles of the present invention. The fabrication process sequence is shown in Figs. 18A-18H. Beginning with a silicon film 1240 on an oxide 1242 on a substrate 1244 (Fig. 18A), the silicon is patterned into device regions as shown in Fig. 18B. Next, a deep implant of an n-type dopant 1241 is performed for producing an n-doped collector region 1250. Referring to Fig. 18C, the device region is doped with boron or other p-type dopants 1243 for providing a p-type base region 1251. Referring to Fig. 18D, the silicon is doped with an n-type dopant 1244 to provide an n+ emitter region 1245. Next, the silicon is heavily doped with an n-type material 1247 to provide an n+ collector region 1248 (Fig. 18E).

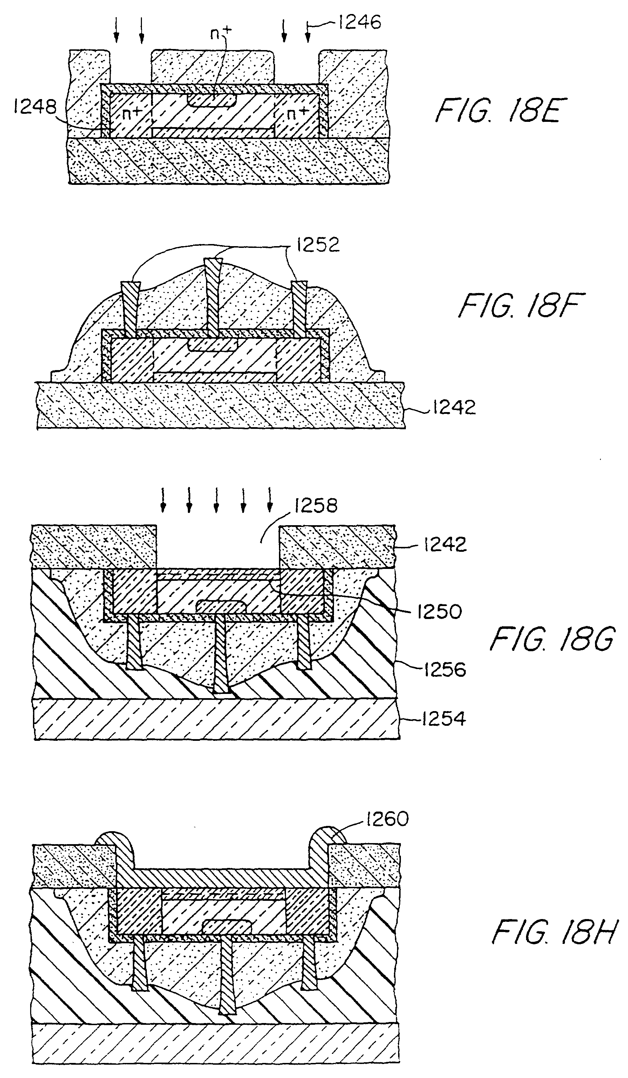

The collector, emitter and base contacts 1252 can be formed (Fig. 18F) and the device can be transferred to a superstrate 1254 (Fig. 18G). The device is attached to the superstrate with an epoxy 1256 and inverted for further processing. To that end, a portion of the oxide layer 1242 is etched forming an opening 1258 at the back of the silicon layer. Next, a metal layer 1260 is applied over the exposed backside of the silicon film and sintered (Fig. 18H). A high temperature implant (∼450°C) can be implemented prior to metalization to produce an n+ buried conductor layer 1250 provided that a high temperature epoxy is used.

Three dimensional circuits can also be fabricated in III-V semiconductor materials, with appropriate release layer material and etchant. For an AlGaAs/GaAs device, an AlAs release layer is preferred. For an InP device, an InGaAs release layer is preferred. AlAs is preferentially etched by HF acid, while InGaAs is preferentially etched by sulfuric/hydrogen peroxide and water solution. The process can also be extended to II-VI semiconductor circuits.

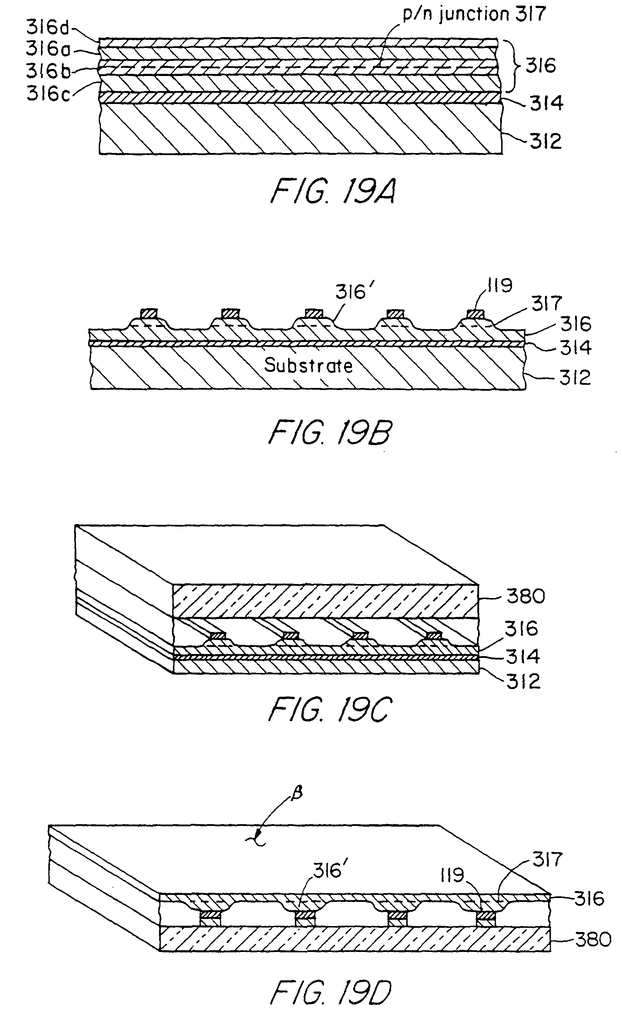

For example, the fabrication of III-V circuit array, in accordance with the invention, will be described with reference to Figs. 19A-D. It begins with the epitaxial growth of the required heteroepitaxial layers of AlGaAs and GaAs layers on a GaAs or Ge substrate. In the case of the GaAs substrate 312, an optional layer 314 of AlAs is formed between the active AlGaAs layers 316 and the substrate 312 to facilitate substrate removal by the etch-off method. The AlAs forms an etch stop layer. Alternatively, the X-Y array can be removed from the substrate by a CLEFT process (See U.S.-A-4,727,047 issued February 23, 1988 to Fan et al.) or chemical epitaxial lift-off. In the case of Ge substrates, a layer of AlAs can be used as an etch stop, but AlAs is not really necessary, since the Ge substrate can be dissolved in H₂O₂ without harm to the AlGaAs active layers. Fig 19A shows the epitaxial layer structure to comprise an active GaAs (or AlGaAs) layer 366 formed by OMCVD. A pattern of contact pads 119 and busbars (not shown) is formed by photolithographic techniques, evaporation, and/or electroplating on the front surface, as shown in Fig. 19B. Next, the circuits 313 are isolated by etching part way into the epitaxial layers 316, as shown in Fig. 19B. This step is not absolutely required at this point, however, it simplifies a later etch step in the process.

The next stage of the process consists of bonding of the wafer to a support 380, such as glass, ceramic, or thin stainless steel. If the support is transparent to infrared radiation, downstream front-to-back alignments are facilitated, but the alignments can also be carried out by careful registration to the support edges. The processed front side is bonded to the support 380 using a suitable adhesive (not shown) (Fig. 19C). After the support 380 is attached, the wafer or substrate 312 is etched off (or cleaved off) leaving the thin film 316 attached to the support 380, as shown in Fig. 19D, in which the structure has been flipped over onto the support to expose the backside B for processing.

Once the backside is exposed, any remaining non-essential material is removed from the back by selective etching in HF to expose a clean GaAs contact layer B. The backside contacts 321 and busbars 321x are now photolithographically patterned and electroplated or evaporated onto the contact regions 316'.

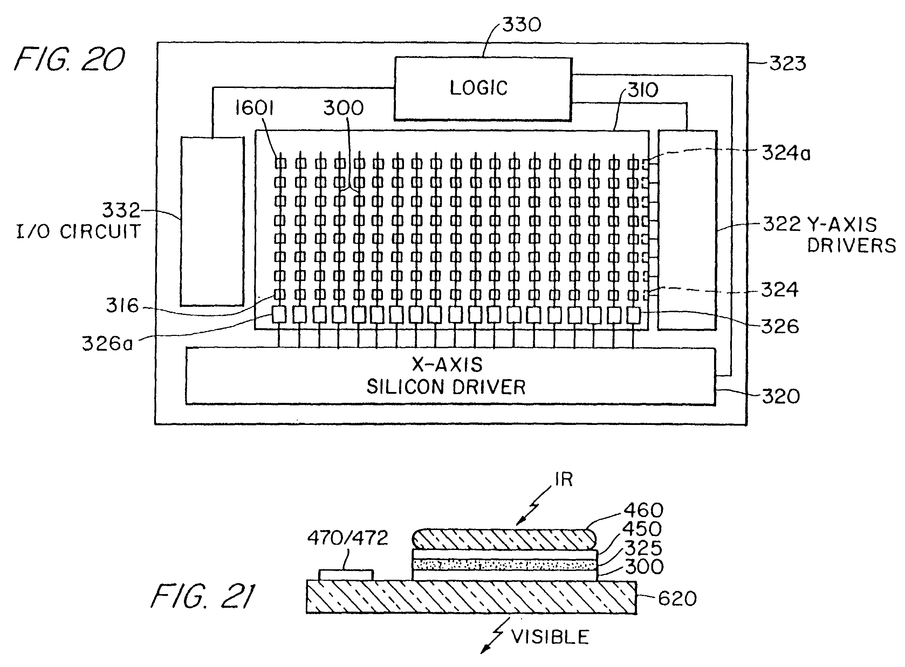

As shown in Fig. 20, the front backside processed circuit array 330 may be mounted directly to a silicon wafer 323 in a precise location 310 with X and Y silicon driver circuits 320 and 322 formed in wafer 323 and coupled to the X and Y bonding pads 324 and 326, respectively. Bonding of array 330 to wafer 323 may also be accomplished by having the contact pads 326 replaced by cantilevered bars that extend over to pads on wafer 323 and which can be trimmed to form circuit bonding pads.

Note that in the first step of the backside process, undesired epitaxial layers are removed; these layers are present to initiate the epitaxy, or may be buffer layers that are not needed in the final device. To make their removal simple, as AlAs etch stop layer (not shown) may be provided in the epitaxy between these layers and the epitaxial device structure. The layers can then be removed in etches that stop at AlAs, such at the well known PA etches. At a pH of about 8, these etches dissolve GaAs 1000 times faster than AsGaAs. After the etch stops at the AlAs, the AlAs can be removed in HF or HCl.

In the process described above, the backside of the substrate is provided with multiplex-compatible metallization to contact the back of each pixel. Note that this type of processing requires front-to-back alignment. The pixels are then separated by a mesa etch. Because the films are only about 5 microns thick, the mesa etch is straightforward and quick. The etching may be accomplished with either wet or dry processing. At this point, the exposed semiconductor may be coated with dielectric to prevent association.

As shown in Fig. 21 a detector 450 and LED array 300 can be stacked into a hybrid assembly comprised of a top thin film IR X-Y detector array 450 affixed by light transparent glue to lower thin film LED array 300 mounted on glass substrate 620. A glass lens 460 is affixed to the top surface of detector 450 and heat transfer openings 460 provided as necessary for cooling purposes. The entire structure can be quite thin (1 mil), with the electronics 470 provided around the periphery. Ultimately, the monolithic thin film array can be mounted on ordinary glasses for image enhancement of visible light, as well as for display of data superimposed on video images.

The applications of the device of Fig. 21 include military night vision systems, -range finders, advanced military avionics, personal communications systems, and medical systems in which real-time image enhancement is useful.

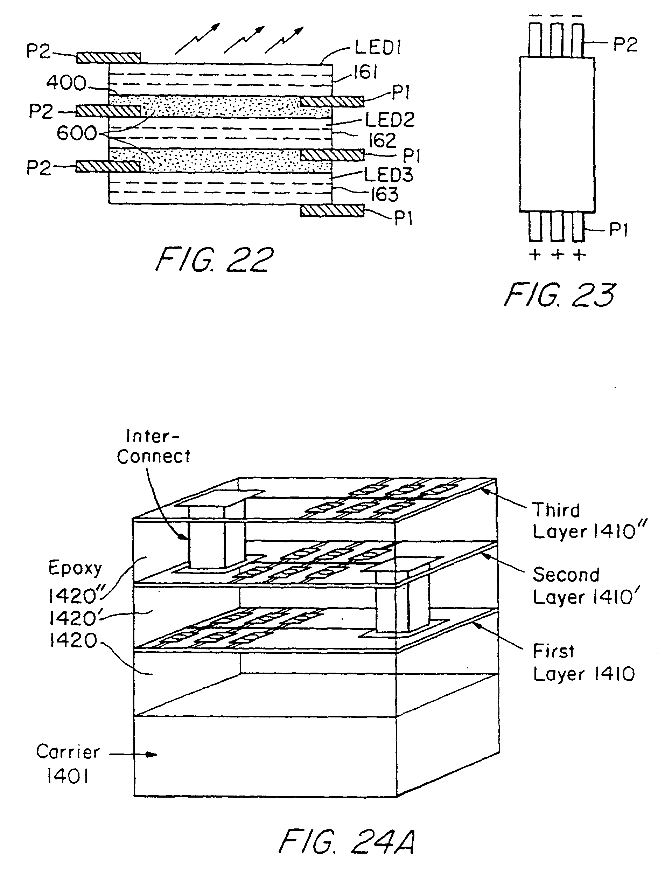

As shown schematically in Figs. 22 and 23, X-Y arrays can also be used to form a multicolor display. To make such a display, individual X-Y arrays labelled LED1, LED2 and LED3, are formed from two or more different epitaxial structure. The primary difference the structure is in the active layer material 161, 162 and 163, which must have different band gaps to create different colors. For example, red 163 can be created with AlGaAs, and green 162 can be created with InAsGaP. The top device LED1 may be a blue LED formed of II-VI material, such as ZnSe, ZnSSe or a group IV alloy such as SiC.

The arrays must be stacked with the larger bandgap LED1 closer to the observer. The material with the larger bandgap will be transparent to the radiation from the smaller bandgap. Thus, in this way, the observer will be able to see both colors.

The creation of the stack of three LEDs 1020 is as follows: first, the three separate LED arrays LED1, LED2 and LED3 are formed, as previously described. Next, they are stacked together with glass 600 between them.

Transparent glue or epoxy 400 is used to bond the stacks on top of each other. The upper and lower bonding pads P1 and P2 on each LED are laterally staggered with respect to other LEDs, so that individual LED pixels may be addressed (See plan view Fig. 23).

Integrated circuits fabricated on other materials can also be stacked into 3D circuit modules using a transfer process as described above. The circuits can be fabricated in II-VI or I-VIII compounds or in diamond thin films. In addition, a 3D circuit module may comprise stacked layers of differing materials. For example, GaAs circuits can be stacked adjacent to Si circuits

Fig. 24A illustrates a 3D circuit stack interconnected by conductive material. The circuits layers 1410, 1410', 1410'' are stacked onto a carrier substrate 1401. The circuit layers 1410, 1410', 1410'' are fabricated separatedly using an above-described transfer process. The circuit layers 1410, 1410', 1410'' are adhered to the carrier substrate 1401 and to adjacent circuit layers by interleaved thin-film epoxy layers 1420, 1420', 1420''. As each layer is transferred interconnects 1414 are formed by patterning and etching the epoxy layers 1420', 1420'' to form vias and then depositing a thin-film metallization layer into the vias. To promote thermal conductivity, away from the circuitry the epoxy may be impregnated or supersaturated with a thermal conductive material, such as diamond.

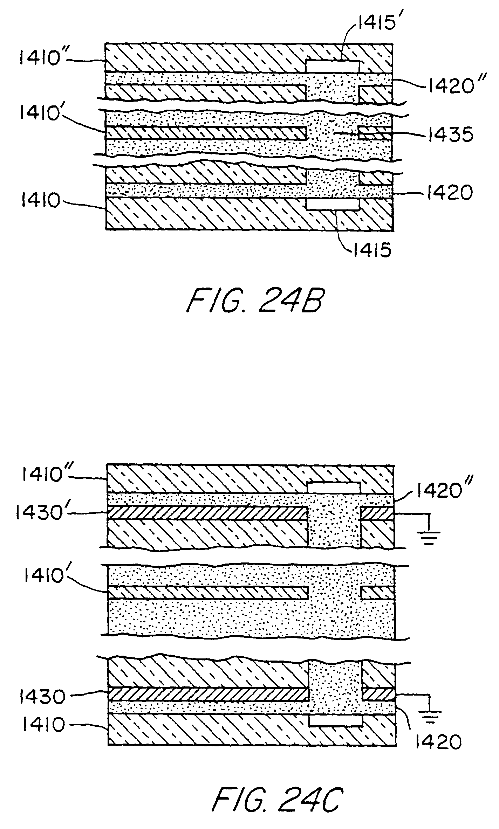

In addition to metal contacts through vias, stacked layers of circuits may employ contactless interconnects. Fig. 24B is a schematic diagram of stacked circuit layers comprising contactless interconnects. Shown are two thin film circuit layers 1410, 1410'' interconnected within a thin film circuit module having a plurality of thin film circuit layers. Each interconnected circuit layer 1410, 1410'' comprises a contactless coupling element 1415, 1415' registered to each other. The plurality of circuit layers are transferred and adhered by interleaved epoxy layers 1420, 1420', 1420''.

Any intervening circuit layer 1410' must be patterned and etched to form vias through that layer. Preferably, the intervening circuit layer 1410' is patterned and etched prior to transfer. The via 1435 is registered to the coupling elements 1415, 1415'. The vias 1435 are filled by epoxy. The intervening layer may also be coupled to the via 1435 by a coupling element (not shown) to create a chain of interconnected circuit layer.

In a preferred embodiment of the invention, the contactless coupling elements 1415, 1415' are transferred LED and detector pairs. In this embodiment, the electrical signals are encoded, optically transmitted to the respective circuit layer, where the optical signal is decoded. The receivers may be transferred patterned fine resolution photovoltaics. The epoxy 1420, 1420', 1420'' must be at least partially transmissive to wavelength of light being transmitted by the LED's. A single circuit layer 1410 may transmit to another particular layer within a chain at interconnected layers utilization of LEDs with various wavelengths chosen to match various detectors.

In alternate preferred embodiment of the invention, shown in Fig 24C. the circuit layers 1410, 1410' are capacitive-coupled by the contactless coupling elements 1415, 1415'. In this embodiment, the coupling elements 1415, 1415' are metallic plates and the epoxy layers 1420, 1425 are diamond impregnated to function as a dielectric separating the two plates. Similarly, the circuit layers 1410, 1410' may be inductive-coupled, where the coupling elements 1415, 1415' are inductive loops. These embodiment are particularly useful in monolithic microwave integrated circuits (MMICs). To limit stray electromagnetic fields from the coupling elements from affecting adjacent circuitry metallic sheilding layers 1430, 1435 are formed in the structure through which the vias 1435 extend.

### D. Thermal Management

An advantage of fabricating stacked 3D circuit modules according to the subject invention is that thermally conductive layers can be inserted between stacked circuit layers to enhance heat transfer to an external heat sink. These layers can also increase the dielectric strength of the intercircuit insulators. The thermally conductive layers are fabricated as thin film layers. The thermally conductive layers may comprise thin film diamond, Sic, AlN, Aluminum, Zr, Ceramics or BeO. The choice of thermally conductive material is dependent on the engineering application.

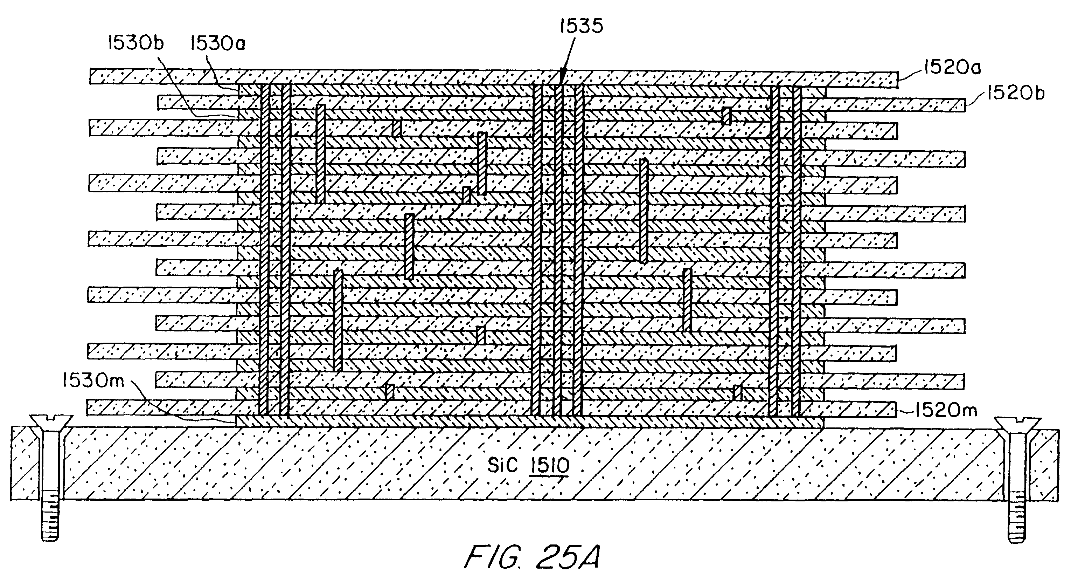

Figure 25A is a schematic diagram of a thin film module having thermally conductive layers. The stacked module has been transferred to a SiC carrier substrate 1510. The module comprises interleaved layers of thermally conductive material 1520a-m and circuit layers 1530a,m. The thin film circuit layers 1530a-m are interconnected by substrate vias 1535.

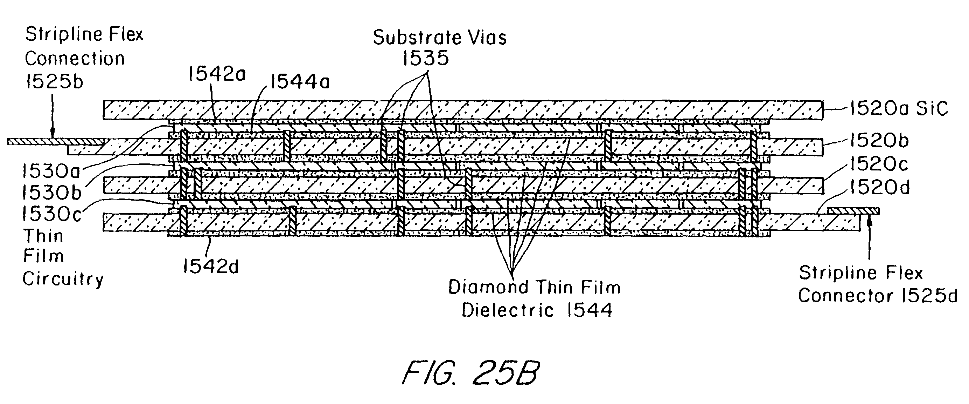

Figure 25B is an exploded view of a section of the stacked circuit module Figure 25A. The thermally conductive layers 1520 comprise SiC. Stripline flex connectors 1535b and 1525d function to dissipate themal energy from the thermally conductive layers 1520b and 1525d to a heat sink. The thin film circuitry 1530 is isolated from the thermally conductive layers 1520 by a diamond thin film dielectric layer 1542, 1544. Preferably the dielectric layer 1542, 1544 is a diamond inpregnated epoxy. The thickness of the dielectric layers 1542, 1544 is determined by the diamond grit diameter, which is preferably about 5 microns.

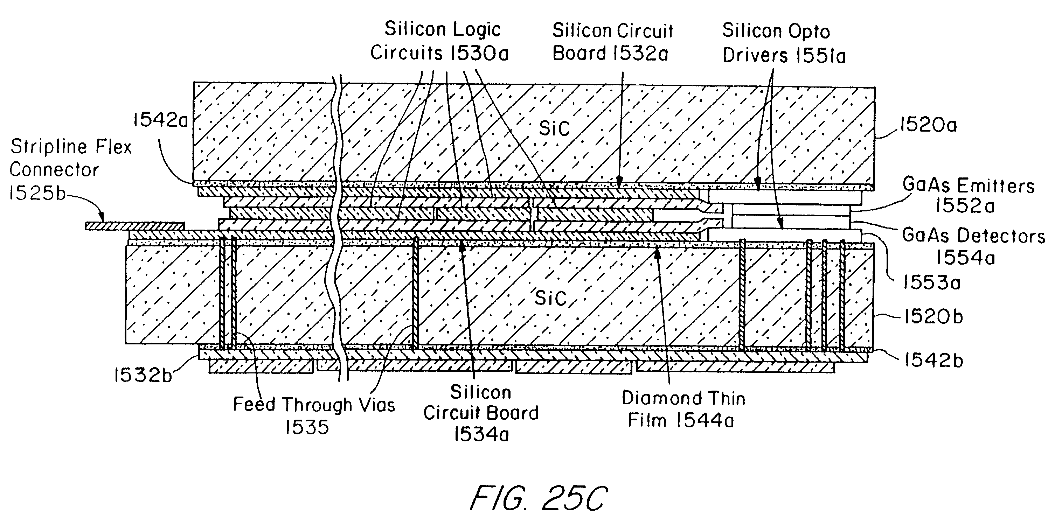

Figure 25C is an exploded view of the thin film module layers of Figure 25B. In particular, the circuit layer interconnect system is detailed. In an preferred embodiment of the invention, an optical interconnect system is used. Fabricated in the circuit layers 1530 are GaAs emitters 1552 and detectors 1554. The emitters are driven by respective optical drivers 1551, 1553.

The stacked thin film semiconductor layers described herein have thicknesses within the range of 0.1 micron to 10 microns and preferably between 0.25 micron and 1.0 micron.

While the invention has been particularly shown and described with reference to preferred embodiments thereof, it will be understood by those skilled in the art that various changes in form and details may be made therein within scope of the present invention which is as defined by the appended claims.

## Claims

1. A method of fabricating a three dimensional circuit module, comprising the steps of:
forming a first circuit layer (1202) on a first substrate (1206);
forming a second circuit layer (1214) on a second substrate;
transferring the second circuit layer (1214) from the second substrate onto the first circuit layer (1202) with an intermediate adhesive layer (1222) separating the first and second circuit layers, said intermediate adhesive layer also adhering both circuit layers together; and transferred forming openings through the intermediate adhesive layer and
interconnecting (1226) the first and second circuit layers through the intermediate layer (1222).

2. The method of Claim 1 wherein the step of transferring comprises:
bonding the second circuit layer (1214) with an adhesive layer to the first circuit layer (1202).

3. The method of Claim 1 wherein the step of transferring comprises:
forming a first adhesive layer (1220) over the second circuit layer (1214);
transferring the second circuit layer (1214) to a superstrate (1218);
forming a second adhesive layer (1222) under the second circuit layer (1214);
transferring the second circuit layer (1214) and the second adhesive layer (1222) onto the first circuit layer (1202); and
solidifying the second adhesive layer (1222) to form the intermediate layer.

4. The method of Claim 1 wherein the step of interconnecting comprises:
forming a via (1226) between the first and second circuit layers; and
coupling the first and second circuit layers through the via (1226).

5. The method of Claim 4 wherein the step of coupling comprises filling the via (1226) with an electrically conduction material (1228).

6. The method of Claim 4 wherein the step of coupling comprises forming an optical link through the via (1226).

7. The method of Claim 4 wherein the step of coupling comprises forming an electromagnetic field link through the via (1226).

8. The method of Claim 1 wherein the intermediate layer (1222) comprise a thermal conductor, the method further comprising the step of interconnecting the intermediate layer to a heat sink (1525).

9. The method of claim 1 wherein the intermediate layer (1222) comprises an electrical conductor, the method further comprising the step of interconnecting the intermediate layer to an electrical ground.

10. The method of claim 1 further comprising removing the first substrate (1206) from the first circuit layer (1202).

11. The method of claim 1 further comprising bonding a third substrate to the second circuit layer (1214).

12. The method of claim 1, comprising positioning the first and second circuit layers (1410) over a transparent substrate; each circuit layer having a plurality of transistors in a thin film semiconductor layer formed on a thin film insulating layer; and
second circuit layer being attached to the first circuit layer without an intervening semiconductor substrate and having a thickness within the range of 10⁻⁷ m to 10⁻⁵ m.

## Patentansprüche

1. Verfahren zum Herstellen eines dreidimensionalen Schaltmoduls, das die Schritte umfasst:
Ausbilden einer ersten Schaltungsschicht (1202) auf einem ersten Substrat (1206),
Ausbilden einer zweiten Schaltungsschicht (1214) auf einem zweiten Substrat,
Übertragen der zweiten Schaltungsschicht (1214) vom zweiten Substrat auf die erste Schaltungsschicht (1202), wobei eine dazwischenliegende Klebeschicht (1222) die erste und zweite Schaltungsschicht trennt, die dazwischenliegende Klebeschicht auch beide Schaltungsschichten zusammenklebt, und
nach dem Übertragungsschritt Ausbilden von Öffnungen durch die dazwischenliegende Klebeschicht und
Verbinden (1226) der ersten und zweiten Schaltungsschichten durch die dazwischenliegende Schicht (1222) hindurch.

2. Verfahren nach Anspruch 1, bei dem der Übertragungsschritt umfasst:
Verbinden der zweiten Schaltungsschicht (1214) mittels einer Klebeschicht mit der ersten Schaltungsschicht (1202).

3. Verfahren nach Anspruch 1, bei dem der Übertragungsschritt umfasst:
Ausbilden einer ersten Klebeschicht (1220) über der zweiten Schaltungsschicht (1214),
Übertragen der zweiten Schaltungsschicht (1214) auf eine Überschicht (superstrate) (1218),
Ausbilden einer zweiten Klebeschicht (1222) unter der zweiten Schaltungsschicht (1214),
Übertragen der zweiten Schaltungsschicht (1214) und der zweiten Klebeschicht (1222) auf die erste Schaltungsschicht (1202), und
Verfestigen der zweiten Klebeschicht (1222), zur Ausbildung der dazwischenliegenden Schicht.

4. Verfahren nach Anspruch 1, bei dem der Verbindungsschritt umfasst:
Ausbilden eines Durchgangs (1226) zwischen der ersten und der zweiten Schaltungsschicht, und
Koppeln der ersten und zweiten Schaltungsschicht durch den Durchgang (1226).

5. Verfahren nach Anspruch 4, bei dem der Kopplungsschritt das Füllen des Durchgangs (1226) mit einem elektrischen Leitungsmaterial (1228) umfasst.

6. Verfahren nach Anspruch 4, bei dem der Kopplungsschritt das Bilden einer optischen Verbindung durch den Durchgang (1226) umfasst.

7. Verfahren nach Anspruch 4, bei dem der Kopplungsschritt das Bilden einer Verbindung durch elektromagnetische Felder durch den Durchgang (1226) umfasst.

8. Verfahren nach Anspruch 1, bei dem die dazwischenliegende Schicht (1222) einen Wärmeleiter umfasst, wobei das Verfahren ferner den Schritt des Verbindens der dazwischenliegenden Schicht mit einem Kühlkörper (1525) umfasst.

9. Verfahren nach Anspruch 1, bei dem die dazwischenliegende Schicht (1222) einen elektrischen Leiter umfasst, wobei das Verfahren ferner den Schritt des Verbindens der dazwischenliegenden Schicht mit einer elektrischen Erde umfasst.

10. Verfahren nach Anspruch 1, welches ferner das Entfernen des ersten Substrats (1206) von der ersten Schaltungsschicht (1202) umfasst.

11. Verfahren nach Anspruch 1, welches ferner das Verbinden eines dritten Substrats (1206) mit der zweiten Schaltungsschicht (1214) umfasst.

12. Verfahren nach Anspruch 1, welches das Positionieren der ersten und zweiten Schaltungsschichten (1410) über einem transparenten Substrat umfasst,
wobei jede Schaltungsschicht eine Vielzahl von Transistoren in einer Dünnfilm-Halbleiterschicht aufweist, welche auf einer Dünnfilm-Isolierschicht ausgebildet ist und
die zweite Schaltungsschicht an der ersten Schaltungsschicht ohne ein vermittelndes Halbleitersubstrat angebracht ist und eine Dicke im Bereich von 10⁻⁷ m bis 10⁻⁵ m hat.

## Revendications

1. Procédé de fabrication d'un module de circuit en trois dimensions, comprenant les étapes :
de formation d'une première couche (1202) de circuit sur un premier substrat (1206) ;
de formation d'une deuxième couche (1214) de circuit sur un deuxième substrat ;
de transfert de la deuxième couche (1214) de circuit depuis le deuxième substrat sur la première couche (1202) de circuit avec une couche adhésive intermédiaire (1222) séparant les première et deuxième couches de circuit, ladite couche adhésive intermédiaire adhérant aussi aux deux couches de circuit ensemble ; et, après l'étape de transfert,
de formation d'ouvertures à travers la couche adhésive intermédiaire, et
d'interconnexion (1226) des première et deuxième couches de circuit par l'intermédiaire de la couche intermédiaire (1222).

2. Procédé selon la revendication 1, dans lequel l'étape de transfert comprend :
la liaison de la deuxième couche (1214) de circuit avec une couche adhésive à la première couche (1202) de circuit.

3. Procédé selon la revendication 1, dans lequel l'étape de transfert comprend :
la formation d'une première couche adhésive (1220) sur la deuxième couche (1214) de circuit ;
le transfert de la deuxième couche (1214) de circuit à un superstrat (1218) ;
la formation d'une deuxième couche adhésive (1222) sous la deuxième couche (1214) de circuit ;
le transfert de la deuxième couche (1214) de circuit et de la deuxième couche adhésive (1222) sur la première couche (1202) de circuit ; et
la solidification de la deuxième couche adhésive (1222) pour former la couche intermédiaire.

4. Procédé selon la revendication 1, dans lequel l'étape d'interconnexion comprend :
la formation d'une traversée (1226) entre les première et deuxième couches de circuit ; et
le couplage des première et deuxième couches de circuit par l'intermédiaire de la traversée (1226).

5. Procédé selon la revendication 4, dans lequel l'étape de couplage comprend le remplissage de la traversée (1226) avec un matériau électriquement conducteur (1228).

6. Procédé selon la revendication 4, dans lequel l'étape de couplage comprend la formation d'une liaison optique par l'intermédiaire de la traversée (1226).

7. Procédé selon la revendication 4, dans lequel l'étape de couplage comprend la formation d'une liaison à champ électromagnétique par l'intermédiaire de la traversée (1226).

8. Procédé selon la revendication 1, dans lequel la couche intermédiaire (1222) comprend un conducteur thermique, le procédé comprenant en outre l'étape d'interconnexion de la couche intermédiaire à un puits de chaleur (1525).

9. Procédé selon la revendication 1, dans lequel la couche intermédiaire (1222) comprend un conducteur électrique, le procédé comprenant en outre l'étape d'interconnexion de la couche intermédiaire à une masse électrique.

10. Procédé selon la revendication 1, comprenant en outre le retrait du premier substrat (1206) de la première couche (1202) de circuit.

11. Procédé selon la revendication 1, comprenant en outre la liaison d'un troisième substrat à la deuxième couche (1214) de circuit.

12. Procédé selon la revendication 1, comprenant le positionnement des première et deuxième couches (1410) de circuit sur un substrat transparent, chaque couche de circuit comportant une pluralité de transistors dans une couche de semiconducteur à film mince formée sur une couche isolante à film mince ; et
la deuxième couche de circuit étant fixée à la première couche de circuit sans l'intervention de substrat semiconducteur et ayant une épaisseur comprise dans la plage de 10⁻⁷ m à 10⁻⁵ m.
